(19) **Europäisches Patentamt**
**European Patent Office**
**Office européen des brevets**

(11) **EP 4 304 447 B1**

(12) **EUROPÄISCHE PATENTSCHRIFT**

(45) Veröffentlichungstag und Bekanntmachung des Hinweises auf die Patenterteilung:
**10.09.2025  Patentblatt 2025/37**

(21) Anmeldenummer: **22714168.6**

(22) Anmeldetag: **11.03.2022**

(51) Internationale Patentklassifikation (IPC):
**A61B 3/028** (2006.01)   **A61B 3/103** (2006.01)
**G02C 7/02** (2006.01)

(52) Gemeinsame Patentklassifikation (CPC):
**A61B 3/028; A61B 3/04; A61B 3/103**

(86) Internationale Anmeldenummer:
**PCT/EP2022/056357**

(87) Internationale Veröffentlichungsnummer:
**WO 2022/189642 (15.09.2022 Gazette 2022/37)**

(54) **VERFAHREN, VORRICHTUNG UND COMPUTERPROGRAMMPRODUKT ZUM BESTIMMEN EINER SENSITIVITÄT ZUMINDEST EINES AUGES EINES PROBANDEN**

METHOD, DEVICE, AND COMPUTER PROGRAM PRODUCT FOR DETERMINING A SENSITIVITY OF AT LEAST ONE EYE OF A TEST SUBJECT

PROCÉDÉ, DISPOSITIF ET PRODUIT PROGRAMME D'ORDINATEUR DE DÉTERMINATION D'UNE SENSIBILITÉ D'AU MOINS UN OEIL D'UN SUJET DE TEST

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.03.2021  DE 102021202442**

(43) Veröffentlichungstag der Anmeldung:
**17.01.2024  Patentblatt 2024/03**

(73) Patentinhaber: **Rodenstock GmbH**
**80687 München (DE)**

(72) Erfinder:
• **TRUMM, Stephan**
**80999 München (DE)**
• **MUSCHIELOK, Adam**
**81476 München (DE)**
• **BECKEN, Wolfgang**
**82061 Neuried (DE)**
• **BÉNARD, Yohann**
**81541 München (DE)**
• **SEIDEMANN, Anne**
**81375 München (DE)**

(74) Vertreter: **Müller-Boré & Partner**
**Patentanwälte PartG mbB**
**Friedenheimer Brücke 21**
**80639 München (DE)**

(56) Entgegenhaltungen:
**EP-B1- 2 499 534     US-A1- 2020 241 320**

**Beschreibung**

Die Erfindung betrifft ein Verfahren, eine Vorrichtung und ein Computerprogrammprodukt zum Bestimmen einer Sensitivität zumindest eines Auges eines Probanden.

Unter der Sensitivität eines Auges wird die Abhängigkeit des Visus dieses Auges von einer Fehlrefraktion verstanden. Die Fehlrefraktion ist hierbei eine Abweichung von einer für das und die Augen eigentlich idealen Refraktion. Mit anderen Worten beschreibt die Sensitivität, wie stark sich der Visus verändert, wenn sich eine vor das Auge vorgeschaltete optische Korrektion ändert.

Die Sensitivität eines Auges oder beider Augen eines Probanden kann beim Berechnen und/oder Erstellen von individuellen Brillengläsern berücksichtigt werden, insbesondere beim Erstellen von Mehrstärkenbrillengläsern wie ophthalmischen Brillengläsern. Brillengläser können Übergänge zwischen Bereichen mit unterschiedlichen optischen Korrektionen aufweisen, also z.B. Übergänge zwischen einem Durchblickspunkt für die Ferne und einem Durchblickspunkt für die Nähe. Gerade diese Übergänge zwischen Brillenglasbereichen mit unterschiedlichen optischen Korrektionen können unterschiedlich ausgestaltet werden. Man spricht hierbei zum Beispiel von harten Übergängen oder von weichen Übergängen, je nachdem wie stark oder sanft die Änderung der Refraktion entlang des Übergangs ausfällt. Bei hochindividuellen und qualitativ hochwertigen Brillengläsern kann insbesondere ein solcher Übergang (aber andere Bereiche des Brillenglases) auf die Sensitivität des oder der Augen des Brillenträgers eingestellt werden.

Zum Erstellen hochindividueller und qualitativ hochwertiger Brillengläser ist somit die Kenntnis der Sensitivität hilfreich. Herkömmlich wird die Sensitivität dadurch gemessen, dass nach einer Bestimmung der subjektiven Refraktion zunächst der Visus bei der ermittelten subjektiven Refraktion ermittelt wird. Anschließend wird und zusätzlich ein weiteres Mal der Visus bestimmt bei einer Refraktion, welche um einen vorgegebenen Abstand von genau 0,5 Dioptrien von der ermittelten subjektiven Refraktion abweicht. Damit ist die Messung eines zweiten Visuswertes abhängig von dem ermittelten subjektiven Refraktionswert. Aus den so ermittelten beiden Visuswerten bei den beiden unterschiedlichen Refraktionswerten kann die Sensitivität ermittelt werden.

Das Dokument EP 2 499 534 B1 beschreibt Verfahren zum Bereitstellen eines Brillenglases für einen Träger, wobei das Verfahren die folgenden aufeinander folgenden Schritte umfasst: Messen des Sehschärfewertes, VA des Auges des Trägers oder des binokularen Sehschärfewertes, VAbino, beider Augen des Trägers, wobei das Auge des Trägers im Wesentlichen frei von Aberrationen niedriger Ordnung ist oder von Aberrationen niedriger Ordnung korrigiert ist; Berechnen eines Designs des Brillenglases mithilfe von Computermitteln oder Auswählen eines Designs in einer Brillenglas-Designdatenbank durch Anpassen der Behandlung des Restastigmatismus basierend auf dem gemessenen Sehschärfewert des Auges des Trägers.

Das Dokument US 2020/0241320 A1 beschreibt ein Verfahren zur Bewertung einer Brillenlinse für einen bestimmten Träger anhand eines Sehleistungsparameters, umfassend die Bereitstellung von Trägerdaten für den bestimmten Träger. Das Verfahren umfasst außerdem die Bereitstellung eines Toleranzbereichs für Sehleistungsparameter für den Träger. Das Verfahren umfasst ferner das Bereitstellen einer zu bewertenden Brillenlinse, wobei die Brillenlinse durch optogeometrische Merkmale charakterisiert ist. Das Verfahren umfasst weiterhin das Berechnen eines Wertes des Sehleistungsparameters für die zu bewertende Linse auf der Grundlage eines Modells. Das Verfahren umfasst außerdem die Bewertung der Brillenlinse durch Vergleichen des berechneten Werts des Sehleistungsparameters mit dem Toleranzbereich des Sehleistungsparameters.

Der Erfindung liegt die Aufgabe zugrunde, das Bestimmen einer Sensitivität zu verbessern und/oder zu vereinfachen.

Diese Aufgabe wird durch die Gegenstände der unabhängigen Ansprüche gelöst. Bevorzugte Ausführungsformen sind die Gegenstände der abhängigen Ansprüche.

Ein Aspekt betrifft ein Verfahren zum Bestimmen einer Sensitivität zumindest eines Auges eines Probanden. Dabei wird eine Bestimmung eines subjektiven Refraktionsergebnisses für das zumindest eine Auge des Probanden durchgeführt. Während des Durchführens der Bestimmung des subjektiven Refraktionsergebnisses wird ein erster Visus des zumindest einen Auges für eine erste angelegte Refraktion bestimmt. Weiterhin wird während des Durchführens der Bestimmung des subjektiven Refraktionsergebnisses ein zweiter Visus des zumindest einen Auges für eine zweite angelegte Refraktion bestimmt. Dabei ist die zweite angelegte Refraktion von der ersten angelegten Refraktion verschieden. Schließlich wird die Sensitivität des zumindest einen Auges unter Berücksichtigung des ersten und des zweiten Visus bei der ersten und der zweiten angelegten Refraktion ermittelt.

Bei dem Verfahren müssen die einzelnen Verfahrensschritte nicht unbedingt in der voranstehend aufgelisteten Reihenfolge durchgeführt werden. Dies bedeutet, dass die einzelnen Verfahrensschritte entweder in der aufgelisteten Reihenfolge, in einer anderen Reihenfolge und/oder auch zumindest teilweise gleichzeitig durchgeführt werden können.

Bei dem Verfahren werden die zumindest zwei benötigten Visuswerte für die zwei unterschiedlichen angelegten Refraktionen bereits während der Durchführung der Bestimmung des subjektiven Refraktionsergebnisses ermittelt. Die Bestimmung des subjektiven Refraktionsergebnisses kann dabei grundsätzlich auf eine herkömmliche und/oder bekannte Art und Weise durchgeführt werden. Bei einer solchen Bestimmung des subjektiven Refraktionsergebnisses wird der Brechwert derjenigen optischen Korrektion bestimmt, mit der das oder die Augen eines Probanden ein scharfes Bild eines zum Beispiel in der Ferne befindlichen Sehobjekts erzeugt oder erzeugen. Diese Bestimmung kann abgekürzt auch als "Durchführung einer subjektiven Refraktion" bezeichnet werden. Zur Durchführung der subjektiven Refraktion gibt es standardisierte Verfahren. Diese Verfahren werden von einem Refraktionisten wie zum Beispiel einem Optiker oder einem Augenarzt durchgeführt. Dazu werden herkömmlicherweise Messvorrichtungen wie eine Probierbrille, Testgläser und-und/oder ein Phoropter verwendet. Die Messvorrichtungen können entweder manuell oder elektrisch vom Refraktionisten bedient werden.

Bei der Bestimmung des subjektiven Refraktionsergebnisses ist der subjektive Seheindruck des Probanden entscheidend für die Bestimmung der benötigten optischen Korrektion. Dabei kommuniziert der Proband mit dem Refraktionisten, indem er ihm eine Rückmeldung gibt bezüglich einer ihm gestellten Sehaufgabe. Die Bestimmung des subjektiven Refraktionsergebnisses kann bei dem erfindungsgemäßen Verfahren auch teilautomatisch oder vollautomatisch durchgeführt werden.

Herkömmlich wird bei der Bestimmung der Sensitivität zunächst das subjektive Refraktionsergebnis ermittelt und der Visus bei dieser subjektiven Refraktion bestimmt, d.h. bei einer optischen Korrektion mit Refraktionswerten, welche dem subjektiven Refraktionsergebnis entsprechen. Anschließend wird die dem oder den Augen vorgestaltete optische Korrektion um einen vorgegebenen Abstand verändert und bei dieser veränderten und somit für den Probanden schlechteren Refraktion ein weiteres Mal der Visus bestimmt. Dies bedeutet, dass bei dem herkömmlichen Verfahren die Bestimmung der Sensitivität erst nach dem Ermitteln des subjektiven Refraktionergebnisses erfolgen kann.

Bei dem erfindungsgemäßen Verfahren hingegen werden jedoch bereits mindestens zwei unterschiedliche Visuswerte während des Ermittelns des subjektiven Refraktionsergebnisses bestimmt. Dadurch kann die Sensitivität bereits während der subjektiven Refraktion ermittelt werden und das Bestimmungsverfahren beschleunigt werden. Mit dem Verfahren kann zumindest der Zusatzschritt der zusätzlichen Visusvermessung bei vorgegebenen Refraktionsabstand eingespart werden; denn der zusätzliche Visus wird nicht erst nach dem Auffinden der subjektiv besten Refraktion bei einer oder mehreren definierten Nebelungen ermittelt, sondern bereits mindestens einmal während des Refraktionsverlaufs erfasst.

Der erste und/oder zweite Visus kann z.B. dadurch bestimmt werden, dass dem Probanden Optotypen angezeigt werden. Der Visus ist abhängig davon, welche Optotypen der Proband bei der eingestellten, angelegten Refraktion erkennen kann.

Nach dem Abschluss der Bestimmung des subjektiven Refraktionsergebnisses kann auf Basis der gemessenen ersten und zweiten Visuswerte und der ersten und zweiten Refraktionswerte die Sensitivität ermittelt werden. Dazu kann insbesondere der Abstand der ersten angelegten Refraktion von dem ermittelten subjektiven Refraktionsergebnis berücksichtigt werden und/oder der Abstand der zweiten angelegten Refraktion von dem ermittelten subjektiven Refraktionsergebnis. Da beim Bestimmen des ersten Visus und/oder des zweiten Visus das subjektive Refraktionsergebnis noch nicht bekannt ist, ist auch der Abstand der ersten angelegten Refraktion oder der zweiten angelegten Refraktion von dem subjektiven Refraktionsergebnis beim Ermitteln des ersten und/oder zweiten Visus noch nicht bekannt.

Während bei dem herkömmlichen Verfahren zur Bestimmung der Sensitivität ein fester Abstand von dem subjektiven Refraktionsergebnis verwendet wird, um einen Visuswert bei diesem festen Abstand zu ermitteln, ist das Verfahren frei von der Nutzung eines fest vorgegebenen Abstandes von dem subjektiven Refraktionsergebnis. Es wird somit die Sensitivität berechnet auf Basis zweier Visuswerte, die bei weitestgehend willkürlich ausgewählten zwei unterschiedlichen Refraktionen ermittelt worden sein können.

Die erste und die zweite angelegte Refraktion erfüllen jedoch zumindest die Mindestbedingung, dass sie in der Sphäre und/oder im Zylinder voneinander abweichen. Die Abweichung beträgt dabei zumindest eine Vierteldioptrie und/oder eine Verdrehung der Zylinderachse um zumindest 45°. Eine Vierteldioptrie ist beim Bestimmen der subjektiven Refraktion üblicherweise der kleinstmögliche Schritt von einer vorgehaltenen optischen Korrektion zur nächsten. Genauso werden dabei üblicherweise die Zylinderachsen in 45°-Schritten verdreht. Die Verwendung dieses Minimalabstands ist notwendig dafür, dass sich die erste Refraktion auch tatsächlich von der zweiten Refraktion unterscheidet. Grundsätzlich kann dieser Minimalabstand bereits ausreichend sein, um die Sensitivität zu bestimmen. Bevorzugt unterscheidet sich die erste Refraktion stärker von der zweiten Refraktion, was nachfolgend näher ausgeführt ist.

Bei der Bestimmung des subjektiven Refraktionsergebnisses werden unterschiedliche optische Korrektionen, also Refraktionen, verwendet, welche dem Probanden angezeigt werden. Die angelegten Refraktionen können dabei grundsätzlich beliebige vorgehaltene optische Wirkungen aufweisen, die noch nicht auf das oder die Augen des Probanden abgestimmt sein müssen. Dabei kann die Visusbestimmung einfach in den Ablauf der Bestimmung der subjektiven Refraktion integriert werden. So kann beispielsweise das subjektive Refraktionsergebnis unmittelbar mit Hilfe von Optotypen bestimmt werden. Abhängig von der Größe und/oder dem Abstand der Optotypen vom Probanden und/oder der aktuell verwendeten optischen Korrektion kann somit der Visus einfach im Laufe der Bestimmung der subjektiven Refraktion ermittelt werden. Dabei können zwei Visuswerte, also zum Beispiel der erste Visus und der zweite Visus, bereits ausreichend sein, um die Sensitivität zu bestimmen. Es können im Laufe der Bestimmung der subjektiven Refraktion allerdings auch mehr als diese zwei Visuswerte bestimmt werden. Dadurch kann die so ermittelte Sensitivität genauer ermittelt werden.

Beim Berechnen der Sensitivität aus den während der Bestimmung der subjektiven Refraktion ermittelten Daten kann zunächst ein erster Abstand der ersten angelegten Refraktion von der bestimmten subjektiven Refraktion ermittelt werden und/oder ein zweiter Abstand der zweiten Refraktion von der bestimmten subjektiven Refraktion. Dieser Abstand kann zumindest bei einem der beiden Visuswerte Null sein, d.h. dass die erste Refraktion oder die zweite Refraktion auch der bestimmten subjektiven Refraktion entsprechen kann. Das Verfahren enthält somit zumindest auch die Möglichkeit, den Visus bei der bestimmten subjektiven Refraktion zu ermitteln und bei der Berechnung der Sensitivität diesen Visuswert bei der subjektiven Refraktion zu verwenden und/oder berücksichtigen.

Das Verfahren kann die Bestimmung der Sensitivität beschleunigen und/oder und vereinfachen. Dies gilt sowohl für den Refraktionisten als auch für den Probanden, welcher nicht mehr nach dem Ermitteln der für ihn günstigsten optischen Korrektion eine zusätzliche Visusprüfung machen muss, um den Visus bei einer vorbestimmten Fehlrefraktion zu ermitteln.

Im Rahmen dieser Anmeldung können die Begriffe Visus und Visuswert synonym verwendet werden, genauso wie die Begriffe Refraktion und/oder Refraktionswert und/oder optische Korrektion. Der Begriff subjektives Refraktionsergebnis kann synonym zum Begriff subjektive Refraktion benutzt werden. Die Begriffe erste und zweite angelegte Refraktion können synonym zu den Begriffen erste und zweite Refraktion, optische Korrektion und/oder angelegte optische Wirkung verwendet werden.

Gemäß einer Ausführungsform wird der erste Visus bestimmt, bevor das subjektive Refraktionsergebnis für das zumindest eine Auge bestimmt ist. Bei dieser Ausführungsform unterscheidet sich die erste Refraktion von der während des Verfahrens bestimmten subjektiven Refraktion. Dabei kann die erste angelegte Refraktion, für welche der erste Visus bestimmt wird, grundsätzlich einen beliebigen Abstand haben von der bestimmten subjektiven Refraktion. Da das Verfahren ohne einen fest vorgegebenen Abstand der ersten Refraktion von der subjektiven Refraktion auskommt, ist der Abstand der ersten Refraktion von der subjektiven Refraktion grundsätzlich nicht vorgegeben, genauso wenig wie der Abstand zwischen der ersten und zweiten Refraktion sowie der Abstand der zweiten Refraktion von der subjektiven Refraktion. Deswegen kann als die erste Refraktion und/oder zweite Refraktion eine Refraktion mit einem jeweils zufälligen Abstand zur subjektiven Refraktion gewählt werden.

Gemäß einer Weiterbildung der Ausführungsform wird als die zweite angelegte Refraktion das bestimmte subjektive Refraktionsergebnis verwendet und der zweite Visus für das bestimmte subjektive Refraktionsergebnis bestimmt. Die Ermittlung der subjektiven Refraktion kann sich hierbei überschneiden und/oder zeitgleich durchgeführt werden mit der Bestimmung des zweiten Visuswertes. Damit liegt zum Ermitteln der Sensitivität einmal der Visus bei der bestimmten subjektiven Refraktion vor, welche als die zweite Refraktion verwendet wird, und weiterhin der erste Visus bei der davon abweichenden ersten Refraktion. Damit liegen grundsätzlich ausreichende Daten vor, um die Sensitivität des zumindest einen Auges des Probanden zu berechnen.

Gemäß einer Ausführungsform wird die Sensitivität des zumindest einen Auges auf Basis einer Sensitivitätsmetrik ermittelt, und diese Ermittlung der Sensitivität erfolgt aus Visusmessungen bei angelegten Refraktionswerten, welche untereinander und/oder vom Refraktionsergebnis keinen speziell zur Ermittlung der Sensitivität vorgegebenen und/oder optimierten Abstand aufweisen. Hierbei ist die Ermittlung der Sensitivität unabhängig von einer Visusmessung bei einem vorgegebenen Abstand von angelegten Refraktionswerten untereinander und/oder vom Refraktionsergebnis. Vielmehr werden die während des Refraktionsvorgangs anfallenden Refraktionswerte und dazugehörenden Visusmessungen sowie ggf. zusätzlich das Refraktionsergebnis zur Sensitivitätsbestimmung genutzt. Bei herkömmlichen Refraktionsverfahren werden diese Refraktions- und Visuswerte mit Ausnahme des Refraktionsergebnisses gar nicht erst bestimmt, da dabei nur letzteres von Interesse ist. Bei herkömmlichen Verfahren zur Refraktionsbestimmung wird die Sensitivität dadurch bestimmt, dass man nach Bestimmung des Refraktionsergebnisses mindestens eine weitere Visusmessung bei einer sich vom subjektiven Refraktionsergebnis unterscheidenden angelegten Refraktion durchführt. Diese sich unterscheidende angelegte Refraktion weist einen vorgegebenen Abstand vom subjektiven Refraktionsergebnis auf. Die Ausführungsform ermöglicht es jedoch, zunächst keine zusätzliche Messung durchführen zu müssen, da durch die Verwendung einer Sensitivitätsmetrik selbst dann eine Sensitivität berechnet werden kann, wenn die angelegten Refraktionswerte und/oder das subjektive Refraktionsergebnis keinen vorgegebenen Abstand voneinander aufweisen.

Die Sensitivitätsmetrik ist hierbei eine Abstandsfunktion, welche zwei Refraktionswerten einen Wert zuordnet, der als Abstand dieser beiden Refraktionswerte voneinander definiert wird. Die Sensitivitätsmetrik kann im metrischen Raum der Refraktionswerte definiert sein. Jedem Refraktionswert der Sensitivitätsmetrik kann ein Visuswert zugeordnet sein. Die Refraktion kann zum Beispiel in einem zumindest dreidimensionalen Raum definiert sein. So kann ein Refraktionswert üblicherweise mit den Koordinaten s, $c$ und a beschrieben werden. Dabei kann s abhängig sein von der Stärke einer optischen Korrektion der Sphäre, c von der Stärke einer optischen Korrektion für einen Zylinder, und a von der Achslage dieses Zylinders. In diesem metrischen Raum der Refraktionswerte werden zumindest die Visuswerte für die erste Refraktion und die zweite Refraktion bestimmt und sind deswegen bei der Berechnung der Sensitivität bekannt. Die Sensitivitätsmetrik kann dazu verwendet werden, die Sensitivität in Abhängigkeit von zwei grundsätzlich beliebigen unterschiedlichen Refraktionswerten zu bestimmen. Durch die Verwendung einer solchen Sensitivitätsmetrik wird die Bestimmung der Sensitivität unabhängig von Visusmessungen bei vorgegebenen Refraktionswerten, wie dies bei herkömmlichen Verfahren üblich ist. Dabei kann die Bestimmung der Sensitivität einerseits unabhängig werden von Visusmessungen bei zumindest einem vorbestimmten und/oder fest vorgegebenen Refraktionsabstand vom Refraktionsergebnis, und andererseits von Visusmessungen bei zumindest einem vorbestimmten und/oder fest vorgegebenen relativen Refraktionsabstand zwischen den zwei angelegten Refraktionen. Damit kann es sowohl für den Refraktionsnisten als auch für den Probanden einfacher werden, die zur Sensitivitätsbestimmung notwendigen Messdaten zu ermitteln.

Sollte die so bestimmte Sensitivität noch nicht mit genügend hoher Genauigkeit bestimmt worden sein, was anhand eines vorgegebenen Schwellenwerts für die Genauigkeit der Sensitivitätsbestimmung festgestellt werden kann, kann gemäß einer weiteren Ausführungsform zusätzlich zu den bereits vorhandenen und während des Refraktionsvorgangs angefallenen Refraktionswerten und/oder dem Refraktionsergebnis samt dazugehörenden Visusmessungen eine oder mehrere zusätzliche Visusmessung(en) bei Refraktionswerten durchgeführt werden, welche vom Refraktionsergebnis einen vorgegebenen und für die Sensitivitätsbestimmung besonders günstigen Abstand besitzen. Ein solcher besonders günstiger Abstand der Refraktionswerte kann sich an der Messgenauigkeit der Refraktion orientieren und z.B. ein Vielfaches dieser betragen (z.B. das 1, 1.5, 2, 3 oder 4-fache der Messgenauigkeit gemessen als dioptrischer Abstand). Die Refraktionswerte werden dabei vorzugsweise so gewählt, dass eine Akkommodation der gemessenen Person während der Messung zu einer Verschlechterung der Sehschärfe führt, insbesondere also dass die zusätzliche(n) Visusmessung(en) bei positiveren Refraktionswerten als dem Refraktionsergebnis stattfindet bzw. stattfinden. Als positivere Refraktionswerte können Refraktionswerte verstanden werden, deren sphärisches Äquivalent, oder vorteilhafter deren beide Hauptschnitte (Sphäre + Zylinder und Sphäre - Zylinder) größer sind als die entsprechenden aus dem Refraktionsergebnis gebildeten Größen.

Gemäß einer Ausführungsform werden zur Bestimmung des subjektiven Refraktionsergebnisses unterschiedliche Optotypen angezeigt, und während der Bestimmung des subjektiven Refraktionsergebnisses wird ein zu den angezeigten Optotypen gehöriger Visus als der erste und/oder zweite Visus bestimmt. Auf diese Art und Weise kann die Visusbestimmung einfach und reibungslos in die Bestimmung der subjektiven Refraktion integriert werden. Hierbei kann die Bestimmung eines Visuswertes bei einer Refraktion, welche von der subjektiven Refraktion abweicht, nahezu automatisch im Rahmen der Bestimmung der subjektiven Refraktion erfolgen. Der so ermittelte Visuswert kann einfach aufgeschrieben und/oder abgespeichert werden zur späteren Verwendung bei der Ermittlung der Sensitivität. Die Verwendung der Optotypen ermöglicht es auch, mehr als nur den ersten oder zweiten Visuswert bei der Bestimmung der subjektiven Refraktion zu ermitteln und/oder aufzuschreiben und/oder abzuspeichern. Durch eine Berücksichtigung von mehr als zwei Visuswerten kann die Ermittlung der Sensitivität weiter verbessert werden. Hierbei kann der zu den Optotypen gehörige Visuswert bestimmt werden unter Berücksichtigung des Abstandes der angezeigten Optotypen von dem Auge des Probanden und/oder der optischen Korrektion sowie von der Größe der angezeigten Optotypen. Die Bestimmung der Visuswerte ist grundsätzlich bekannt und wird deswegen an dieser Stelle nicht näher ausgeführt.

Gemäß einer Ausführungsform weist die erste angelegte Refraktion einen Abstand von der zweiten angelegten Refraktion von zumindest einer halben Dioptrie in der Sphäre und/oder von zumindest einer Dioptrie eines Zylinders auf. Ein solcher Mindestabstand führt zu besonders guten Ergebnissen bei der Ermittlung der Sensitivität. Deswegen werden bevorzugt die Visuswerte bei zwei Refraktionen verwendet, die einen solchen Mindestabstand in der Sphäre und/oder im Zylinder aufweisen.

Bei einer Ausführungsform wird nach dem Bestimmen des subjektiven Refraktionsergebnisses überprüft, ob die erste angelegte Refraktion einen vorbestimmten sphärischen und/oder zylindrischen Mindestabstand von der zweiten angelegten Refraktion aufweist. Für den Fall, dass dieser vorbestimmte Mindestabstand unterschritten ist, wird ein dritter Visus für eine dritte angelegte Refraktion bestimmt, welche zumindest um den vorbestimmten Mindestabstand von der ersten und/oder zweiten angelegten Refraktion beabstandet ist. Die Sensitivität des zumindest einen Auges wird unter Berücksichtigung des dritten Visus bei der dritten angelegten Refraktion ermittelt. Hierbei kann die Sensitivität auch unter Berücksichtigung aller drei Visuswerte ermittelt werden. Auch wenn in diesem Ausführungsbeispiel eine zusätzliche Visusmessung der Bestimmung der subjektiven Refraktion nachgeschaltet ist, so bietet diese Ausführungsform dennoch die Möglichkeit, die Bestimmung der Sensitivität zu vereinfachen und/oder zu verkürzen. Dies gilt nämlich insbesondere für den Fall, dass die erste Refraktion den vorbestimmten sphärischen und/oder zylindrischen Mindestabstand von der zweiten Refraktion einhält. Der vorgegebene Mindestabstand kann beispielsweise eine halbe Dioptrie in der Sphäre und/oder eine ganze Dioptrie im Zylinder sein. Denn für den Fall, dass der Mindestabstand zwischen der ersten und zweiten Refraktion vorliegt, kann auf die Bestimmung der dritten Refraktion verzichtet werden. Deswegen bietet dieses Verfahren immerhin noch die Option auf eine verkürzte und/oder vereinfachte Sensitivitätsbestimmung. Diese Verkürzung und/oder Vereinfachung wird durch die der subjektiven Refraktionsbestimmung nachgelagerte Überprüfung des Mindestabstands ermöglicht.

Gemäß einer Ausführungsform wird die Sensitivität des zumindest einen Auges auf Basis eines linearen Modells ermittelt, bei welchem eine Abhängigkeit der Sensitivität für einen zylindrischen Refraktionsabstand von der Sensitivität für einen sphärischen Refraktionsabstand angenommen wird. Wird diese Annahme getroffen, also dass die Sensitivität für einen zylindrischen Refraktionsabstand von der Sensitivität für einen sphärischen Refraktionsabstand abhängt, so kann die Sensitivität bereits auf Basis der zwei Visuswerte bei den beiden unterschiedlichen Refraktionen ermittelt werden. Dieses lineare Modell stellt ein vereinfachtes Modell dar. Hierbei wird ein Zusammenhang zwischen dem sphärischen und zylindrischen Refraktionsabstand angenommen, welcher sich in der Abhängigkeit der Sensitivitäten wiederspiegelt. Deswegen ist dieses lineare Modell einer Sensitivitätsmetrik besonders vorteilhaft, wenn lediglich die zwei Visuswerte ermittelt werden. Dadurch wird die Bestimmung der Sensitivität stark vereinfacht.

Gemäß einer Ausführungsform wird ein dritter Visus des zumindest einen Auges für eine dritte angelegte Refraktion bestimmt. Die Sensitivität des zumindest einen Auges wird unter Berücksichtigung des ersten, zweiten und dritten Visus bei der ersten, zweiten und dritten angelegten Refraktion auf Basis eines bilinearen Modells ermittelt. Das bilineare Modell der Sensitivitätsmetrik ist etwas komplexer als das voranstehend erwähnte lineare Modell. Es benötigt für die Berechnung der Sensitivität zumindest drei Visuswerte bei drei unterschiedlichen Refraktionen. Bei dieser Ausführungsform unterscheidet sich somit die erste Refraktion sowohl von der zweiten Refraktion als auch von der dritten Refraktion. Weiterhin unterscheidet sich auch die zweite Refraktion von der dritten Refraktion. Vereinfacht gesagt unterscheiden sich alle drei Refraktionswerte voneinander. Dadurch werden drei Messwerte des Visus in der Sensitivitätsmetrik zur Verfügung gestellt, auf Basis derer die Sensitivität ermittelt werden kann. Dies verbessert die Genauigkeit der Ermittlung der Sensitivität gegenüber dem vorab erwähnten linearen Modell. Auch das bilineare Modell basiert auf einer Näherung, liefert aber bereits deutlich bessere Ergebnisse als das lineare Modell. Dabei kann auch der dritte Visus bereits während der Bestimmung der subjektiven Refraktion ermittelt werden. Alternativ kann der dritte Visus erst nach der Bestimmung der subjektiven Refraktion ermittelt werden, z.B. unter einem vorgegebenen Abstand zu der ersten und/oder zweiten Refraktion und/oder der subjektiven Refraktion.

Sowohl das lineare Modell als auch das bilineare Modell der Sensitivitätsmetrik kann empirische Messdaten berücksichtigen, also insbesondere Abhängigkeiten der Sensitivität von der Sphäre, vom Zylinder und/oder von der Achslage. Diese empirischen Daten können weiterhin abhängig vom Alter der Probanden sein, um das lineare und/oder bilineare Modell an das Alter des Probanden anzupassen. Dabei kann die Bestimmung der Sensitivität gegebenenfalls verbessert werden.

Gemäß einer Ausführungsform wird die Sensitivität ohne Berücksichtigung des bestimmten subjektiven Refraktionsergebnisses ermittelt. Es wird somit die Sensitivität nicht unter Berücksichtigung eines Abstandes der ersten und/oder zweiten Refraktion von der subjektiven Refraktion ermittelt, sondern auf Basis des Abstands zumindest zweier Visuswerte voneinander, also z.B. auf Basis des Abstands des ersten Visus vom zweiten Visus. Der Abstand der beiden Visuswerte von der subjektiven Refraktion kann dabei unberücksichtigt bleiben. Auch diese Ermittlung wird durch Verwendung einer geeigneten Sensitivitätsmetrik ermöglicht. Bei dieser Ausführungsform muss insbesondere keine optische Wirkung mit der "bestimmten subjektiven Refraktion" vorgehalten werden, um die Sensitivität zu ermitteln. Bei dieser Ausführungsform kann ggf. das subjektive Refraktionsergebnis aus einem angepassten Modell für die Abhängigkeit des Visus von der vorgesetzten Wirkung entnommen werden.

Gemäß einer Ausführungsform wird das subjektive Refraktionsergebnis nicht anhand, d.h. unabhängig von, einer vorgehaltenen Korrektion beim subjektiven Refraktionsergebnis bestimmt, sondern das subjektive Refraktionsergebnis wird aus einem angepassten Modell für die Abhängigkeit des Visus von der optischen Wirkung der vorgehaltenen Korrektion bestimmt. Das Verfahren kann somit ohne Vorhalten des subjektiven Refraktionsergebnisses durchgeführt werden. Hierzu werden bevorzugt zumindest drei Visuswerte bei zumindest drei unterschiedlichen Korrektionen bestimmt, aus denen z.B. mittels einer geeigneten Sensitivitätsmetrik das subjektive Refraktionsergebnis berechnet werden kann. In diesem Fall kann das Bestimmen des subjektiven Refraktionsergebnisses abgekürzt werden, da es nach einer ausreichenden Anzahl von bestimmten Visuswerten nicht mehr weitergeführt werden muss.

Gemäß einer Ausführungsform wird die Bestimmung des subjektiven Refraktionsergebnisses mittels einer Refraktionseinheit durchgeführt und/oder erfolgt die Visusbestimmung mittels dem Probanden angezeigter Optotypen. Als Refraktionseinheit kann zum Beispiel eine Refraktionsbrille und/oder ein Phoropter verwendet werden. Der Phoropter kann vom Refraktionisten manuell, teilautomatisch und/oder vollautomatisch gesteuert werden. Die Verwendung eines Phoropters in Kombination mit Sehaufgaben auf Basis von dem Probanden angezeigten Optotypen ist hierbei besonders bevorzugt und vereinfacht das Verfahren.

Gemäß einer Ausführungsform erfolgt das Bestimmen der subjektiven Refraktion, das Bestimmen des ersten und zweiten Visus, und/oder das Bestimmen der Sensitivität softwaregestützt und/oder zumindest teilautomatisch. Hierbei können Visuswerte mit den zugehörigen Refraktionswerten automatisch gesteuert, überprüft und/oder zur Bestimmung der Sensitivität weiter verarbeitet werden.

Gemäß einer Ausführungsform wird der erste Visus und/oder der zweite Visus monokolar und/oder binokular bestimmt. Die Entscheidung für einen monokolare und/oder binokulare Visusbestimmung kann abhängig vom Probanden und/oder von zu erstellenden optischen Korrektionen sein.

Gemäß einer Ausführungsform wird die ermittelte Sensitivität dazu verwendet, zumindest ein individuelles Brillenglas für den Probanden zu erstellen. Die ermittelten Messdaten können dazu benutzt werden, das zumindest eine individuelle Brillenglas gut an das zumindest eine Auge des Benutzers anzupassen. Dies gilt insbesondere für das Erstellen von ophthalmischen Brillengläsern.

Gemäß einer Ausführungsform wird zur Bestimmung des subjektiven Refraktionsergebnisses eine Lichtfeldanzeige verwendet, um dem Probanden zumindest ein Prüfbild mit simulierten Wellenfronten anzuzeigen. Die simulierten Wellenfronten können dabei vorgehaltenen optischen Wirkungen entsprechen, also z.B. der ersten und/oder zweiten angelegten Refraktion. Die Lichtfeldanzeige kann im Rahmen der Bestimmung der subjektiven Refraktion ein oder mehrere Prüfbilder mit zugehörigen simulierten angelegten Refraktionen gleichzeitig und/oder nacheinander anzeigen.

Mittels der Lichtfeldanzeige können Sehzeichen (wie z.B. Optotypen) z.B. tabellarisch angezeigt werden. Dabei kann z.B. für jede Spalte (alternativ: für jede Zeile) eine andere angelegte Refraktion simuliert wird und die Sehzeichen z.B. in jeder Zeile (alternativ: in jeder Spalte) eine andere (z.B. nach unten bzw. rechts abnehmende) Größe aufweisen. Dann kann der Proband gefragt werden, bis zu welcher Spalte und/oder Zeile er die Sehzeichen subjektiv gut erkennen und/oder vorlesen kann. So kann der Visus zeitgleich mit der subjektiv besten angelegten Refraktion ermittelt werden. Dies kann eine schnelle Bestimmung des subjektiven Refraktionsergebnisses ermöglichen.

Weiterhin kann der Proband für zwei oder mehrere Spalten und/oder Zeilen gefragt werden, bis zu welcher jeweiligen Zeile und/oder Spalte er die Sehzeichen jeweils gut erkennen kann. Damit können Visus für unterschiedliche angelegte Refraktionen ohne Änderung der Darstellung der Anzeige und/oder im direkten Vergleich bestimmt werden.

Ein Aspekt betrifft eine Vorrichtung zum Bestimmen einer Sensitivität zumindest eines Auges eines Probanden mit einer Refraktionseinheit zum Bestimmen eines subjektiven Refraktionsergebnisses für das zumindest eine Auge des Probanden. Eine Visusbestimmungseinheit ist dazu konfiguriert, während des Durchführens der Bestimmung des subjektiven Refraktionsergebnisses einen ersten Visus des zumindest einen Auges für eine erste angelegte Refraktion zu bestimmen und einen zweiten Visus des zumindest einen Auges für eine zweite angelegte Refraktion. Dabei ist die zweite angelegte Refraktion von der ersten angelegten Refraktion verschieden. Eine Sensitivitätsermittlungseinheit ermittelt die Sensitivität des zumindest einen Auges unter Berücksichtigung des ersten und des zweiten Visus bei der ersten und zweiten angelegten Refraktion.

Die Vorrichtung kann als ein Vorrichtungssystem ausgebildet sein und zusätzliche Einheiten umfassen, beispielsweise eine Anzeigeeinheit und/oder eine Steuereinheit. Weiterhin kann die Vorrichtung und/oder das Vorrichtungssystem auf eine Brillenglasdatenerstellungseinheit umfassen, welche beispielsweise in eine Steuerung integriert sein kann. Eine oder mehrere dieser Einheiten können softwaregesteuert ausgebildet sein. Die Vorrichtung kann dazu benutzt werden, das voranstehend beschriebene Verfahren durchzuführen. Deswegen betreffen sämtliche Ausführungen zum Verfahren auch die Vorrichtung und umgekehrt.

Ein Aspekt betrifft ein Computerprogrammprodukt umfassend computerlesbare Programmteile, welche geladen und ausgeführt eine Vorrichtung nach dem voranstehenden Aspekt dazu bringen, ein Verfahren nach dem eingangs beschriebenen Aspekt durchzuführen, wobei das Computerprogrammprodukt zumindest eine der folgenden Einheiten zumindest teilweise steuert und/oder regelt:

- die Refraktionseinheit;
- die Visusbestimmungseinheit;
- die Sensitivitätsermittlungseinheit;
- eine Steuerung; und/oder
- eine Brillenglasdatenerstellungseinheit zum Erstellen zumindest eines individuellen Brillenglases und/oder zum Berechnen zumindest einer Brillenglasfläche aus den erfassten Messdaten.

Das Computerprogrammprodukt kann zur Teilautomatisierung und/oder Vollautomatisierung der Sensitivitätsbestimmung und/oder der Bestimmung der subjektiven Refraktion verwendet werden.

Die Erfindung wird nachfolgend anhand von Ausführungsbeispielen und Figuren näher beschrieben. Einzelne Merkmale dieser Ausführungsbeispiele und Figuren können mit anderen Ausführungsbeispielen kombiniert werden. Es zeigen

Figur 1A      in einer schematischen Darstellung einen Kreuzzylinder in einer ersten Position;

Figur 1B      in einer schematischen Darstellung einen Kreuzzylinder in einer zweiten Position;

Figur 2      in einer schematischen Darstellung eine erste Ausführungsform einer Vorrichtung zum Bestimmen einer Sensitivität zumindest eines Auges eines Probanden in einem ersten Anzeigezustand;

Figur 3      in einer schematischen Darstellung eine zweite Ausführungsform einer Vorrichtung zum Bestimmen einer Sensitivität zumindest eines Auges eines Probanden in einem zweiten Anzeigezustand;

Figur 4      in einer schematischen Darstellung ein Lichtfelddisplay einer Vorrichtung zum Bestimmen einer Sensitivität; und

Figur 5      in einer schematischen Darstellung ein Lichtfelddisplay einer Vorrichtung zum Bestimmen einer Sensitivität.

Im Rahmen dieser Erfindung können die Begriffe "im Wesentlichen" und/oder "etwa" so verwendet sein, dass sie eine Abweichung von bis zu 5% von einem auf den Begriff folgenden Zahlenwert beinhalten, eine Abweichung von bis zu 5° von einer auf den Begriff folgenden Richtung und/oder von einem auf den Begriff folgenden Winkel.

Begriffe wie oben, unten, oberhalb, unterhalb, lateral, usw. beziehen sich - sofern nicht anders spezifiziert - auf das Bezugssystem der Erde in einer Betriebsposition des Gegenstands der Erfindung.

## Ausführungsbeispiele einer Sensitivitätsmetrik

Die Sensitivität kann mit Hilfe eines metrischen Raums berechnet werden, in welchem unterschiedliche Refraktionswerte einzelne Punkte repräsentieren. Ein Refraktionswert kann z.B. dreidimensional dargestellt werden, z.B. mit den Koordinaten $s, c,$ und $\alpha$. Hierbei kann s von der Stärke einer sphärischen Korrektion abhängen und z.B. in Dioptrie (was auch mit dpt abgekürzt werden kann) angegeben werden. $c$ kann von der Stärke einer zylindrischen Korrektion abhängen und z.B. in dpt angegeben werden. $\alpha$ kann von der Achslage der zylindrischen Korrektion abhängigen und z.B. in Grad angegeben werden, z.B. von 0 bis 180°. Alternativ dazu können andere Koordinaten verwendet werden.

Im Folgenden wird beispielhaft davon ausgegangen, dass die beste Refraktion, also das bestimmte subjektive Refraktionsergebnis, in dieser Sensitivitätsmetrik mit $s_0, c_0,$ und $\alpha_0$ bezeichnet wird und der dazugehörige Visus mit $v_0$. Bei der Durchführung des Verfahrens werden zumindest zwei Visuswerte ermittelt. Im allgemeinen Fall können neben dem Visus $v_0$ bei der subjektiven Refraktion bei n weiteren Refraktionen $s_i, c_i, \alpha_i$ mit dazugehörigem Visus $v_i$ mit $i \in [1, ..., n]$ bestimmt worden sein und somit vorliegen.

In einer möglichen Sensitivitätsmetrik berechnet sich der Abstand einer Refraktion i von der besten Refraktion in der mittleren Sphäre $d_i$ und im Zylinder $a_i$ mit der Gleichung (1) zu:

$$d_i = \left(s_i + \tfrac{1}{2} \cdot c_i\right) - \left(s_0 + \tfrac{1}{2} \cdot c_0\right)$$
$$a_i = \sqrt{c_i^2 + c_0^2 - 2 \cdot c_i \cdot c_0 \cdot \cos(\alpha_i - \alpha_0)} \tag{1}$$

## Einfaches bilineares Modell einer Sensitivitätsmetrik mit Kenntnis der subjektiven Refraktion

In einer Ausführungsform eines bilinearen Modells einer Sensitivitätsmetrik gilt für die Abhängigkeit des Visus der folgende, in Gleichung (2) aufgezeigte Zusammenhang für jede Einzelmessung für eine Refraktion $i$. Dabei kann in einem vereinfachten Fall davon ausgegangen, dass der Proband eine Nebelung nicht durch Akkommodation ausgleichen kann.

$$\lg v_i = m_d \cdot |d_i| + m_a \cdot a_i + \lg v_0 \tag{2}$$

Hierbei steht $m_d$ für die Sensitivität bei einem sphärischen Abstand und $m_a$ für die Sensitivität bei einem zylindrischen Abstand. Eine solche Trennung zwischen einer sphärischen und einer zylindrischen Fehlrefraktion kann verwendet werden, um zu berücksichtigen, dass Probanden sehr unterschiedlich auf diese beiden Komponenten einer Fehlrefraktion reagieren können. So kann aus Daten aus D. Methling: Bestimmung von Sehhilfen, 2. Aufl. Ferdinand Enke Verlag, Stuttgart 1996, ermittelt werden, dass im Bevölkerungsdurchschnitt empirisch ermittelt in etwa die Gleichungen (3) gelten:

$$m_d = \tfrac{2}{1\,\text{dpt}} \cdot \lg 0{,}5 = -0{,}601\,\text{dpt}^{-1}$$
$$m_a = \tfrac{1}{1\,\text{dpt}} \cdot \lg 0{,}5 = -0{,}301\,\text{dpt}^{-1} \tag{3}$$

Im Allgemeinen hat die voranstehende Gleichung (2) drei die unabhängigen Parameter $m_a, m_d, v_0$. Daher kann das Gleichungssystem (2) mit drei Messungen i=1,2,3 von drei Visuswerten $v_1, v_2, v_3$ bei drei unterschiedlichen Refraktionen ($s_1, c_1, \alpha_1; s_2, c_2, \alpha_2; s_3, c_3, \alpha_3$) eindeutig gelöst werden zum Gleichungssystem (2a): *mit*

$$m_a = -\frac{1}{nenner}\left(\log\left(v_1^{|d_2|-|d_3|}\right)+\log\left(v_2^{|d_3|-|d_1|}\right)+\log\left(v_3^{|d_1|-|d_2|}\right)\right)$$

$$m_d = -\frac{1}{nenner}\left(\log\left(v_1^{a_3-a_2}\right)+\log\left(v_2^{a_1-a_3}\right)+\log\left(v_3^{a_2-a_1}\right)\right) \qquad (2a)$$

$$\log v_0 = -\frac{1}{nenner}\left(\log\left(v_1^{a_2|d_3|-a_3|d_2|}\right)+\log\left(v_2^{a_3|d_1|-a_1|d_3|}\right)+\log\left(v_3^{a_1|d_2|-a_2|d_1|}\right)\right)$$

$$nenner = (a_2-a_3)|d_1|+(a_3-a_1)|d_2|+(a_1-a_2)|d_3|$$

Hierbei kann in einer bevorzugten Ausführungsform eine der drei Visusmessungen bei optimalen Korrektionsbedingungen stattfinden, also bei der subjektiven Refraktion. Dann gilt z.B. bei i=3: $(s_3, c_3, \alpha_3)=(s_0, c_0, \alpha_0)$. Bei dieser optimalen Korrektionsbedingung ist dann $a_3 = a_0 = 0$ und $d_3 = d_0 = 0$. Somit ist die dritte der Gleichungen (2a) automatisch erfüllt. Die anderen Gleichungen nehmen dann folgende Form des Gleichungssystems (4) an:

mit

$$m_a = -\frac{1}{nenner}\left(|d_2|\log(v_1/v_0)-|d_1|\log(v_2/v_0)\right)$$

$$m_d = -\frac{1}{nenner}\left(-a_2\log(v_1/v_0)+a_1\log(v_2/v_0)\right) \qquad (4)$$

$$nenner = a_2|d_1|-a_1|d_2|$$

Das Gleichungssystem (4) stellt somit ein Ausführungsbeispiel eines vereinfachten bilinearen Modells einer Sensitivitätsmetrik bereit. Das Gleichungssystem (4) kann unter Kenntnis der subjektiven Refraktion sowie unter Kenntnis zwei zusätzlicher Visuswerte für zwei zusätzliche Refraktionswerte (für i=1,2) gelöst werden. Damit kann aus dem Gleichungssystem (4) die Sensitivität ermittelt werden.

Werden neben dem Visus $v_0$ bei der subjektiven Refraktion mehr als zwei zusätzliche Visuswerte gemessen, kann die Sensitivität genauer ermittelt werden, indem $m_d$ und $m_a$ über ein Ausgleichsverfahren, z.B. der Methode kleinster Quadrate, aus allen Daten bestimmt werden. Weiterhin können Ausreißer aus den Messdaten ausgeschlossen werden, um die Qualität der Sensitivitätsbestimmung zu erhöhen.

**Vereinfachtes lineares Modell einer Sensitivitätsmetrik mit Kenntnis der subjektiven Refraktion**

In einem weiter vereinfachten, weniger individuellen Modell der Sensitivitätsmetrik, z.B. wenn nur eine Messung bei einer Fehlrefraktion i=1 vorhanden ist, kann ein Zusammenhang zwischen dem sphärischen und dem zylindrischen Refraktionsabstand angenommen werden gemäß Gleichung (5):

$$m_d = m$$
$$m_a = f \cdot m_d = f \cdot m \qquad (5)$$

Hierbei kann der Parameter $f$ aus empirischen Werten abgeleitet werden und z.B. ein Skalar sein. Mit der Annahme gemäß Gleichung (5) vereinfacht sich das Gleichungssystem (2) zur folgenden Gleichung (6):

$$\lg v_i = m \cdot (d_i + f \cdot a_i) + \lg v_0 \qquad (6)$$

Damit lässt sich die Sensitivität m aus einer Messung bei einer Fehlrefraktion i ermitteln aus Gleichung (7):

$$m = \frac{a_i + f \cdot |d_i|}{\lg v_i - \lg v_0} \qquad (7)$$

Ein Wert für $f$ aus kann aus einschlägiger Fachliteratur abgeleitet werden, z.B. kann $f = 1/2$ gesetzt werden, abgeleitet aus Applegate, R.A, Sarver, E.J, Khemsara, V., Are all aberrations equal?, J Refract Surg. 2002, 18:S556-S562. Oder es kann $f = 1$ gesetzt werden, abgeleitet aus Atchison et al. 2009, Blur limits for defocus, astigmatism and trefoil, VisionResearch.

Dabei muss für Gleichung (5) nicht unbedingt ein linearer Zusammenhang angenommen werden. Es können alternativ komplexere Zusammenhänge aufgestellt werden und die Sensitivität daraus, z.B. in Abhängigkeit von einer Anzahl der unabhängigen Parameter und/oder der Visusmessungen - durch Einsetzen in entsprechend aufgelöste Zusammenhänge abgeleitet werden, vgl. Gleichungen (4) und (7). Die Sensitivität kann auch aus einem Ausgleichsverfahren, wie z.B. kleinste Quadrate, abgeleitet werden.

**Weitere Modelle einer Sensitivitätsmetrik mit Kenntnis der subjektiven Refraktion**

Die Sensitivität kann auch auf Basis eines anderen Modells berechnet werden. So sind z.B. aus R. Blendowske, Unaided Visual Acuity and Blur: A Simple Model, Optometry and Vision Science, Vol. 92, No. 6, 2015, Modelle bekannt, die sich durch besondere Einfachheit auszeichnen und die auf nur wenigen Parametern basieren. Solche einfachen Modelle sind zur Berechnung der Sensitivität und zur Anpassung bei geringer Datenlage besonders geeignet, beispielsweise weil damit ein Overfitting gut vermieden werden kann.

Ist eine größere Anzahl an Parametern individuell verfügbar, eignet sich ein Modell mit vielen unterschiedlichen Parametern besser, wie es etwa in der Druckschrift DE 10 2017 007 663 A1 beschrieben ist.

Grundsätzlich kann eine Vielzahl unterschiedlicher Modelle verwendet werden. Das im Einzelfall verwendete Modell kann dabei von der Anzahl der ermittelten Visuswerte bei unterschiedlichen Refraktionen abhängen. Bei hinreichend vielen gemessenen Visuswerten können relativ komplexe, nicht notwendigerweise lineare Modelle aufgestellt werden, deren Parameter an die Messungen angepasst werden können.

Die voranstehend beispielhaft aufgezählten Modelle lassen sich verallgemeinern, z.B. indem eine die Sehschärfe beschreibende Funktion im Powervektorraum Konturen konstanter Sehschärfe aufweist, welche den Punkt maximaler Sehschärfe beinhaltenden Ellipsoiden oder Ovoiden entsprechen. Dies kann analog zu einem in A. Rubin und W. F. Harris, Closed Surfaces of Constant Visual Acuity in Symmetric Dioptric Power Space, Optometry and Vision Science, Vol. 78, No. 10, 2001, vorgestellten Verfahren erfolgen. Dabei können sich Achsverhältnisse in einem Bereich von 0.25 bis 4 individuell unterscheiden. Anstatt individuell gemessener Werte können auch Mittelwerte, Mediane oder andere Schätzwerte der entsprechenden Modellparameter der Population zur Berechnung der Sehschärfe verwendet werden.

In einem Ausführungsbeispiel führt eine Verallgemeinerung von der voranstehenden Gleichung (6) zu unterschiedlichen Faktoren f, z.B. zur Gleichung (8):

$$\lg v_i = \left\{ \left( d_i, a_i^{ort}, a_i^{obl} \right) R \begin{bmatrix} m_1^2 & 0 & 0 \\ 0 & m_2^2 & 0 \\ 0 & 0 & m_3^2 \end{bmatrix} R^T \left( d_i, a_i^{ort}, a_i^{obl} \right)^T \right\}^{1/2} + \lg v_0 \tag{8}$$

Dabei bezeichnen $a_i^{ort}$ und $a_i^{ort}$ den Astigmatismus der Fehlrefraktion mit orthogonalen (J0) bzw. schiefen (J45) Achslagen und sind definiert als:

$$a_i^{ort} = -\frac{c_i}{2} \cos(2\alpha_i) + \frac{c_0}{2} \cos(2\alpha_0)$$

$$a_i^{obl} = -\frac{c_i}{2} \sin(2\alpha_i) + \frac{c_0}{2} \sin(2\alpha_0)$$

und

$R$ stellt dabei eine Rotationsmatrix dar, welche eine Orientierung eines Ellipsoids konstanter Sehschärfe im Powervektorraum der Vektoren $\left( d_i, \ a_i^{ort}, a_i^{obl} \right)$ bestimmt. Die Eigenwerte $m_1, m_2, m_3$ bezeichnen die Sensitivitäten gegen-über Nebelung in die Richtung des ersten, zweiten bzw. dritten Spaltenvektors der Rotationsmatrix R im Powervektorraum.

**Ausführungsformen von Modellen einer Sensitivitätsmetrik ohne Kenntnis der subjektiven Refraktion**

In einigen Ausführungsformen kann die Ermittlung der Sensitivität ohne Ermittlung der subjektiven Refraktion durchgeführt werden. Dies kann dann erfolgen, wenn für mehrere unterschiedliche Refraktionen eine Visusmessung durchgeführt wird. In diesem Fall kann die beste Refraktion, also die subjektive Refraktion, aus den dabei anfallenden Messdaten ermittelt werden. Weiterhin kann aus den Messdaten eine tatsächlich ermittelte beste subjektive Refraktion mittels eines Modells einer Sensitivitätsmetrik überprüft werden.

Dabei kann davon ausgegangen werden, dass eine Nebelung, also eine absichtliche Fehlrefraktion, zu Minus hin vom Probanden durch eine Akkommodation des zumindest einen Auges ausgeglichen werden kann. In diesem Fall lässt sich in dem linearen Modell gemäß den voranstehenden Gleichungen (2) und (6) ein Punkt wählen, an dem die Visuskurve abknickt. Bei nicht-linearen Modellen, bei denen sich eine Sättigung ergibt, kann die beste Refraktion direkt als Parameter des Gleichungssystems berechnet werden. Dazu muss in den entsprechenden Formeln, also insbesondere bereits in Gleichung (1), die Fehlrefraktion, d.h. die Abstände $d_i$ und $a_i$, durch die Differenz zwischen bester Refraktion und Korrektion bei der Visusmessung ersetzt werden.

## Ausführungsbeispiel eines Verfahrens zur Bestimmung der subjektiven Refraktion mit Erfassung von Visuswerten

In einem Ausführungsbeispiel wird zunächst eine objektive Refraktion für einen Probanden bestimmt, d.h. es werden Refraktionswerte auf Basis einer objektiven Messung ermittelt. Beispielsweise ist aus der Druckschrift DE 10 2011 120 973 A1 ein Verfahren bekannt, wie solche objektiven Parameter bestimmt werden können. Die objektiven Parameter können aberrometrische Messdaten und/oder pupillometrische Messdaten umfassen. Die objektiv ermittelten Messdaten, also die aberrometrischen Messdaten und/oder die pupillometrischen Messdaten, können zur Berechnung einer objektiv optimierten Refraktion verwendet werden.

Eine Bestimmung der besten subjektiven Refraktion, also des subjektiven Refraktionsergebnisses, kann grundsätzlich mittels eines im Stand der Technik beschrieben Verfahrens erfolgen, vgl. hierzu z.B. D. Methling: Bestimmung von Sehhilfen, 2. Aufl. Ferdinand Enke Verlag, Stuttgart (1996).

Als Ausgangspunkt für die Bestimmung des subjektiven Refraktionsergebnisses können die objektiv ermittelten Refraktionswerte verwendet, welche um einen vorgegebenen Abstand vernebelt werden, z.B. um eine zusätzliche Sphäre von 0,50 bis 1,00 dpt. Diese so vernebelten Refraktionswerte können als Startrefraktionswerte verwendet werden.

Anstelle der objektiv ermittelten Refraktionswerte können andere Refraktionswerte als Startrefraktionswerte verwendet werden, z.B. die Werte eines bereits vorhandenen alten Korrektionsmittels, also z.B. einer älteren Brille. Alternativ können als Startrefraktionswerte beliebige Refraktionswerte und somit beliebige vorgehaltene optische Wirkungen verwendet werden.

Anschließend werden bei der Bestimmung der subjektiven Refraktion des Probanden die folgenden vier Hauptverfahrensschritte a) bis d), gegebenenfalls ergänzt um den Verfahrensschritt e), durchgeführt. Bei einer monokularen Bestimmung der subjektiven Refraktion kann alternativ auch nur der Verfahrensschritt a) oder b) durchgeführt werden, optional ergänzt um Verfahrensschritt e):

a) Monokulare Bestimmung der positivsten sphärisch-zylindrischen Refraktion, bei welcher subjektiv die beste Sehschärfe für ein erstes Auge des Probanden bewirkt wird, z.B. für das rechte Auge;
b) Monokulare Bestimmung der positivsten sphärisch-zylindrischen Refraktion, bei welcher subjektiv die beste Sehschärfe für ein zweites Auge des Probanden bewirkt wird, z.B. für das linke Auge;
c) Einstellen eines binokularen, akkommodativen Gleichgewichts;
d) Binokulare Bestimmung der positivsten sphärisch-zylindrischen Refraktion, bei welcher subjektiv die beste Sehschärfe für beide Augen des Probanden bewirkt werden; und
e) Subjektive Bewertung der so ermittelten Refraktionswerte in einem Testrahmen

Dabei folgen diese Verfahrensschritte einem logischen Muster, welches auf Visusmessungen beruht. Eine Veränderung der Stärke der sphärischen Korrektion erfolgt dabei bevorzugt nur dann, wenn die Zugabe einer Sphäre von -0,25 dpt den Visus, also die Sehschärfe, um eine Linie verbessert, also eine Veränderung von -0,1 logMAR bewirkt. Diese Bedingung kann durchgehend als ein Veränderungskriterium bei der Zugabe einer negativen Sphäre verwendet werden.

Bei einer Veränderung zu einer positiven sphärischen Wirkung hin, also unter Zugabe einer Sphäre von +0,25 dpt, muss der Visus besser werden oder gleichbleiben. Diese Bedingung kann durchgehend als ein Veränderungskriterium bei der Zugabe einer positiven Sphäre verwendet werden.

Diese unterschiedlichen Kriterien ergeben sich daraus, dass man sich bei der Zugabe einer positiven Sphäre auf einem Plateau befinden kann. Startet man z.B. mit +3,00 dpt und beträgt die tatsächlich benötigte Refraktion z.B. +3,25 dpt, so wird sich der Visus nicht ändern, egal ob man +0,25 dpt zugibt oder -0,25 dpt. Deswegen gilt für die Zugabe einer positiven Sphäre das weitere Veränderungskriterium, welches auch für einen dabei gleichbleibenden Visus gilt.

Eine Linie der Sehschärfe kann hierbei als erreicht angesehen werden, wenn der Proband zumindest 60% der angezeigten Optotypen dieser Linie erkennen kann.

Bei der monokularen Bestimmung der Refraktion für das erste Auge, also im **Verfahrensschritt a),** kann zunächst eine Stärke der benötigten Sphärenkorrektion der subjektiven Refraktion bestimmt werden. Dabei kann das zweite Auge abgedeckt werden, also z.B. das linke Auge. Dem ersten Auge wird die gemessene Startrefraktion für das rechte Auge vorgehalten. Falls eine Visusbestimmung für die Startrefraktion einen Visuswert von 0,1 logMAR oder besser ergibt, kann der Startrefraktion eine erste positive Linse für das erste Auge hinzugefügt werden. Anschließend wird der Visus erneut gemessen. Falls dabei das jeweilige Veränderungskriterium erreicht wird, wird solange erneut eine weitere positive Linse hinzugefügt werden, bis das zutreffende Veränderungskriterium nicht mehr erreicht wird. Wenn das zutreffende Veränderungskriterium nicht mehr erreicht wird, kann der Refraktionist stattdessen eine negative Linse zuschalten. Falls dabei das Veränderungskriterium erreicht wird, kann solange erneut eine weitere negative Linse hinzugefügt werden, bis das Veränderungskriterium nicht mehr erreicht wird.

Damit ist die Bestimmung der Stärke der benötigten Sphärenkorrektion abgeschlossen und es folgt eine Bestimmung einer Achse und/oder Achslage einer ggf. benötigten Zylinderkorrektion.

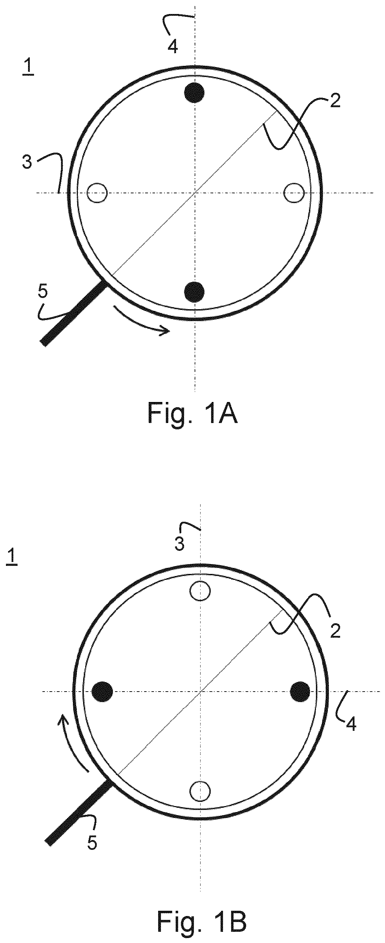

Dazu kann ein Refraktionist einen Kreuzzylinder 1 benutzen, um eine Achse eines ggf. vorhandenen Astigmatismus des ersten Auges zu bestimmen. **Figuren 1A und 1B** zeigen in einer schematischen Darstellung einen solchen Kreuzzylinder 1, welcher auch unter dem Namen "Jackson cross cylinder" und Jackson Kreuzzylinder bekannt ist. Der Kreuzzylinder 1 weist einen Griff 5 auf, durch welchen eine Griffachse 2 verläuft. Der Kreuzzylinder 1 ist ein optisches Hilfsmittel und weist zwei unter 90° gekreuzte Zylinder auf, nämlich einen Pluszylinder und einen Minuszylinder. Die Griffachse 2 ist unter 45° zu einer Zylinderachse 3 des Pluszylinders angeordnet und unter 45° zu einer Zylinderachse 4 des Minuszylinders angeordnet.

Dem Probanden können Optotypen angezeigt werden, die auf eine schlechteste Sehschärfe von zumindest 0,2 logMAR hinweisen. Die Griffachse 2 des Kreuzzylinders 1 kann auf einer vermuteten und/oder der objektiv ermittelten Achse eines Astigmatismus des ersten Augers des Probanden angeordnet werden. Anschließend kann der Kreuzzylinder 1 zwischen den beiden in den Figuren 1A und 1B gezeigten Positionen umgedreht werden, wobei die Griffachse 2 positionsgetreu verbleibt. Der Proband kann gefragt werden, welche dieser beiden Drehpositionen des Kreuzzylinders 1 ein besseres Seherlebnis bewirkt. Stellt der Proband keinen Unterschied fest, ist die Achse für die Refraktion des ersten Auges gefunden und es wird mit einer Bestimmung der benötigten Zylinderstärke weitergemacht, vgl. unten. Falls der Proband eine der beiden in den Figuren 1A und 1B gezeigten Drehpositionen bevorzugt, kann die Griffachse 2 genau dann im Uhrzeigersinn verlagert werden, wenn die Zylinderachse 4 des Minuszylinders in der bevorzugten Drehposition im Uhrzeigersinn von der Griffachse 2 steht, vgl. Situation in Fig. 1B. Die Griffachse 2 kann genau dann im Gegenuhrzeigersinn verlagert werden, wenn die Zylinderachse 4 des Minuszylinders in der bevorzugten Drehposition im Gegenuhrzeigersinn von der Griffachse 2 steht, vgl. Situation in Fig. 1B.

Diese Überprüfung mit den beiden Drehpositionen und dem Verdrehen der Griffachse 2 kann solange wiederholt werden, bis der Proband keinen Unterschied mehr zwischen den beiden Drehpositionen erkennt, oder bis die Griffachse 2 dabei hin- und herbewegt wird. Im letzteren Fall kann diejenige Achse aus diesen zuletzt verwendeten Achslagen ausgewählt werden, welche am ehesten mit einer älteren Achse übereinstimmt, also z.B. mit einer Achse für dieses erste Auge, die in einer älteren Brille des Probanden verwendet wurde. Alternativ kann diejenige Achse aus diesen zuletzt verwendeten Achslagen ausgewählt werden, welche näher an einem nicht-schrägen Astigmatismus angeordnet ist.

Damit ist die Bestimmung der Achse der benötigten Zylinderkorrektion abgeschlossen und es folgt eine Bestimmung einer Stärke der benötigten Zylinderkorrektion.

Der Kreuzzylinder 1 kann dazu so angeordnet werden, dass seine Zylinderachse 3 des Pluszylinders und seine Zylinderachse 4 des Minuszylinders genau auf den entsprechenden Zylinderachsen der objektiv ermittelten Refraktion angeordnet sind, welche dem ersten Auge des Probanden bereits vorgehalten ist. Der Kreuzzylinder 1 kann genauso umgedreht werden wie bei der voranstehend beschriebenen Bestimmung der Achslage, also unter positionsgetreuer Lage der Griffachse 2. Falls eine dabei vom Probanden bevorzugte Drehposition diejenige Drehposition ist, bei der die beiden Zylinderachsen der Minuszylinder überlappen, so kann eine negative Zylinderstärke hinzugefügt werden, z.B. -0,25 dpt. Falls die dabei vom Probanden bevorzugte Drehposition diejenige Drehposition ist, bei der die Zylinderachsen 4 des Pluszylinders des Kreuzzylinders 1 die Zylinderachse des Minuszylinders der vorgehaltenen Refraktion überlappt, so kann eine negative Zylinderstärke weggenommen werden, auch z.B. in Schritten von Vierteldioptrien. Der Refraktionist kann dies solange wiederholen, bis der Proband keine der Drehpositionen mehr bevorzugt, oder bis sich die Stärke der Zylinderkorrektion dabei hin und her verändert. Im letzteren Fall sollte die niedrigste dabei verwendete Stärke der Zylinderkorrektion ausgewählt werden.

Bei der Bestimmung der Stärke der benötigten Zylinderkorrektion kann darauf geachtet werden, dass die vorab ermittelte Stärke der benötigten Sphärenkorrektion gleichbleibt. Dies bedeutet, dass z.B. für jede Veränderung der Stärke der Zylinderkorrektion um 0,50 dpt auch die Stärke der Sphärenkorrektion um 0,25 dpt in die andere Richtung verändert wird.

Nach der Bestimmung sowohl der Stärke als auch der Achse der benötigten Zylinderkorrektion kann noch einmal die Sphärenkorrektion überprüft werden. Dazu kann genauso vorgegangen werden, wie voranstehend im Zusammenhang mit der Bestimmung der Stärke der benötigten Sphärenkorrektion beschrieben. Optional kann für den Fall, dass sich dabei die Stärke stark ändert, die Achsenbestimmung wiederholt werden, um ein zuverlässigeres Ergebnis zu erzielen.

Damit ist eine monokulare Bestimmung der subjektiven Refraktion für das erste Auge abgeschlossen, welche sich aus der bestimmten Stärke der benötigten Sphärenkorrektion und der bestimmten Stärke und Achse der benötigten Zylinderkorrektion zusammensetzt.

Anschließend erfolgt die Bestimmung der Refraktion für das zweite Auge, also der **Verfahrensschritt b).** Dieser erfolgt genau analog zum Verfahrensschritt a), nur für das zweite Auge und bei abgedecktem ersten Auge.

Als Ergebnis wird die monokulare subjektive Refraktion für das zweite Auge bestimmt, welche sich aus einer bestimmten Stärke einer benötigten Sphärenkorrektion und einer bestimmten Stärke und Achse einer benötigten Zylinderkorrektion zusammensetzt.

In **Verfahrensschritt c)** erfolgt ein Einstellen des binokularen, akkommodativen Gleichgewichts. Dazu werden beide Augen aufgedeckt. Den beiden monokularen subjektiven Refraktionen für das erste und zweite Auge kann jeweils eine Vernebelung zugefügt werden, z.B. eine Vernebelung von jeweils +0,50 dpt. Zudem kann ein Separator verwendet werden, also z.B. ein Polarisationsfilter und/oder ein rot/grün-Filter. Ein Ziel dabei kann es sein, dass beide Augen denselben Akkommodationszustand eingehen. Der Separator kann es dem Probanden erlauben, mit jedem seiner Augen unterschiedliche Anzeigeteile einer Zielanzeige zu sehen und deren Schärfe miteinander zu vergleichen. Hierfür sollte zusätzlich zu den Anzeigeteilen, die nur von jeweils einem Auge wahrgenommen werden können, ein gemeinsamer Anzeigeteil der Zielanzeige für beiden Augen sichtbar sein. Erst dann kann eine sinnvolle Fusion stattfinden und die Sehschärfe überprüft werden. Falls eines der beiden Augen schärfer sieht als das andere, kann die Refraktion dieses Auges weiter mit einer positiven Sphäre vernebelt werden. Der Refraktionist kann diesen Vorgang wiederholen, bis beide Anzeigeteile der Zielanzeige dem Probanden etwa gleichscharf erscheinen, oder bis dabei der geringste Schärfenunterschied wahrgenommen wird.

Für den **Verfahrensschritt d),** also für die binokulare Bestimmung der positivsten sphärisch-zylindrischen Refraktion, bei welcher subjektiv die beste Sehschärfe für beide Augen des Probanden bewirkt wird, wird zunächst der Separator entfernt. Anschließend wird eine Visusbestimmung durchgeführt. Für die binokulare Bestimmung der Stärke der benötigten Sphärenkorrektion kann nach demselben Vorgehen wie in den Verfahrensschritten a) und b) vorgegangen werden, nur hier für beide Augen zugleich. Die beiden bereits bestimmten monokularen zylindrischen Refraktionen können hierbei unverändert bleiben. Das Ergebnis kann als die binokulare subjektive Refraktion verwendet werden. Alternativ kann die binokulare subjektive Refraktion als das Ergebnis des Ergänzungsschritts e) bestimmt werden.

Es folgt der **Verfahrensschritt e),** bei welchem eine subjektive Bewertung der nach Verfahrensschritt d) enthaltenen Refraktionswerte in einem Testrahmen erfolgt. Dazu werden die in Verfahrensschritt d) ermittelten Refraktionswerte in einen Testrahmen gegeben und an das Gesicht des Probanden angepasst. Dazu können insbesondere die Pupillen des Probanden in der Mitte von Testlinsen mit diesen Refraktionswerten zentriert werden. Eine Überprüfung der Testlinsen kann in einer offenen Umgebung im Freien erfolgen, wobei der Proband ein Ziel in der Ferne fixieren kann.

Der Refraktionist kann binokular eine Sphärenstärke von +0,25 dpt hinzugeben und den Probanden fragen, ob der Seheindruck mit oder ohne diese Zugabe besser erscheint oder gleichbleibt. Falls der Seheindruck durch diese Zugabe besser erscheinen sollte oder gleichbleibt, so werden als endgültig bestimmte subjektive Refraktionswerte die in Verfahrensschritt d) ermittelten Refraktionswerte verwendet, welche um diese binokulare Zugabe von +0,25 dpt in der Sphäre ergänzt werden.

Falls die Zugabe zu keinem verbesserten oder zumindest gleichbleibenden Seheindruck führt, so kann binokular die Sphärenstärke um -0,25 dpt verändert werden und der Probanden gefragt werden, ob der Seheindruck mit oder ohne diese Reduktion um eine Vierteldioptrie besser erscheint. Falls diese Veränderung ins Negative zu einem besseren Seheindruck führen sollte, so kann binokular nochmals die Sphärenstärke um -0,25 dpt verändert werden. Der Proband kann gefragt werden, ob der Seheindruck bei einer Veränderung von -0,25 dpt oder von -0,50 dpt besser erscheint. Falls die Veränderung um -0,25 dpt zu einem besseren Seheindruck führt, so werden als endgültig bestimmte subjektive Refraktionswerte die in Verfahrensschritt d) ermittelten Refraktionswerte verwendet, welche um die binokulare Veränderung um -0,25 dpt in der Sphäre ergänzt werden. Falls die Veränderung um -0,50 dpt zu einem besseren Seheindruck führt, so werden als endgültig bestimmte subjektive Refraktionswerte die in Verfahrensschritt d) ermittelten Refraktionswerte verwendet, welche um die binokulare Veränderung um -0,50 dpt in der Sphäre ergänzt werden.

Damit ist die Bestimmung der binokularen subjektiven Refraktion für den Probanden beendet.

Während dieser Bestimmung der binokularen subjektiven Refraktion wird der Visus bei mindestens zwei unterschiedlichen vorgeschalteten Refraktionen gemessen. Es wird zumindest ein erster Visus bei einer ersten Refraktion und ein zweiter Visus bei einer zweiten Refraktion gemessen. Dabei kann z.B. der Visus insbesondere bei der besten Korrektion bestimmt werden, d.h. bei den Refraktionswerten, die am Ende von Verfahrensschritt d) bestimmt wurden. Zusätzlich kann der Visus bei einer davon abweichen Korrektion bestimmt werden, bevorzugt bei einer mehr ins Plus verschobenen Korrektion. Dies kann deswegen von Vorteil sein, da mehr Minus vom Probanden eventuell durch Akkommodation ausgeglichen werden kann. Bevorzugt weichen die beiden Korrektionen, bei denen die beiden Visuswerte bestimmt werden, von etwa 0,50 dpt bis etwa 1,25 dpt in der Sphäre voneinander ab.

Der Visus kann bei einer von der bestimmten subjektiven Refraktion abweichenden zylindrischen Korrektion gemessen werden. Dazu können Jackson Kreuzzylinder benutzt werden, z.B. mit plus/minus 0,50 dpt, oder ein plus Zylinder mit z.B. +1,00 dpt im Vergleich zur bestimmten subjektiven Refraktion.

Die Stärke der Abweichung der Korrektion von der optimalen Korrektion kann dabei auch von der Höhe der Addition abhängen oder sich grob nach den Maximalwerten des bei einem Gleitsichtglas erwarteten ungewollten Astigmatismus richten. Der Visus eines Probanden mit einer geringeren Addition würde demnach bei weniger sphärischer bzw. zylindrischer Nebelung gemessen werden als der Visus eines Probanden mit einer höheren Addition.

Der Visus kann mittels Optotypen bestimmt werden, wobei ein Visus als erreicht gilt, wenn mindestens 60% der Optotypen einer zugehörigen Zeile erkannt wurden.

Der Visus kann während der und/oder nach den Verfahrensschritte(n) a), b), c) und/oder d) gemessen und für eine nachfolgende Berechnung der Sensitivität abgespeichert und/oder aufgeschrieben werden. Beispielsweise können während des Verfahrensschritts c) und/oder d) zumindest zwei binokulare Visuswerte ermittelt werden. Es können während der Verfahrensschritte a) und/oder b) ein oder mehrere monokulare Visuswerte ermittelt werden.

Der Visus kann insbesondere während der Verfahrensschritte a) und/oder b) monokular bestimmt werden. Dazu werden nachfolgend bevorzugte Zeitpunkte der Verfahrensschritte a) und b) aufgelistet, an denen eine Visusbestimmung vorteilhaft ist.

Der Visus kann insbesondere am Anfang der Bestimmung der subjektiven Refraktion gemessen werden, also z.B. am Anfang der Verfahrensschritte a) und/oder b), da gerade die zuerst angelegte Refraktion in der Regel noch einen relativ großen Abstand vom subjektiven Refraktionsergebnis aufweist. Ein hinreichender Abstand zwischen der ersten und zweiten angelegten Refraktion führt in der Regel zu einer zuverlässigen Bestimmung der Sensitivität.

Der Visus kann während des Abgleichs der Sphäre bestimmt werden und/oder nachdem die Sphäre ermittelt worden ist. Gerade die angelegte Refraktion beim danach ermittelten Visus unterscheidet sich in der Regel deutlich von der anfangs angelegten Refraktion. Deswegen kann z.B. ein monokularer Visus bei der (in Verfahrensschritt a) und/oder in Verfahrensschritt b)) zuerst und zuletzt angelegten Refraktion bestimmt werden.

Der Visus kann vor der Bestimmung der Achslage der Zylinderkorrektion bestimmt werden, insbesondere genau dann, wenn geprüft wird, ob eine Zylinderkorrektion überhaupt notwendig ist. Dies kann in Verfahrensschritt a) und/oder b ) erfolgen.

Der Visus kann während des Abgleichs der Achse bestimmt werden und/oder nachdem die Achse ermittelt ist. So kann der Visus z.B. bei einer Position des Kreuzzylinders bestimmt werden, oder bei gegeneinander verdrehten Positionen des Kreuzzylinders. Wird der Visus bei gegeneinander verdrehten Positionen des Kreuzzylinders bestimmt, so ist den Visuswerten garantiert ein astigmatischer Unterschied zugeordnet, was wiederum zu einer zuverlässigen Bestimmung der Sensitivität führen kann.

Der Visus kann während des Abgleichs der Zylinderstärke bestimmt werden und/oder nachdem die Zylinderstärke ermittelt ist.

Analog zu den voranstehend ausgeführten Beispielen, wann der Visus während der Verfahrensschritte a) und b) bestimmt werden kann, kann der Visus auch binokular während der Verfahrensschritte c) und d) bestimmt werden. Hierbei kann er insbesondere ganz am Anfang der Verfahrensschritte c) und/oder d) und/oder ganz am Ende dieser Verfahrensschritte bestimmt werden.

Bevorzugt wird der Visus mehr als zweimal bestimmt, also zumindest dreimal oder gar zumindest viermal. Dabei erhöht die Anzahl der Visusmessungen die Genauigkeit der Sensitivitätsermittlung. Die Anzahl der Visusmessungen kann insbesondere gerade dann die Genauigkeit erhöhen, wenn sich die zugeordneten angelegten Refraktionen nur gering voneinander unterscheiden.

Deswegen kann in einer Ausführungsform z.B. überprüft werden, wie stark sich die erste und zweite angelegte Refraktion voneinander unterscheiden. Ist der Unterschied zu gering, so wird der Visus zumindest ein drittes Mal bestimmt für eine dritte angelegte Refraktion, welche sich sowohl von der ersten als auch von der zweiten unterscheidet.

Generell kann als Prüfbild immer, d.h. bei jeder angelegten Refraktion, ein Bild angezeigt werden, anhand dessen der Visus bestimmt werden kann. So können auf dem Prüfbild z.B. unterschiedlich große Sehzeichen wie z.B. Optotypen in unterschiedlichen Spalten und/oder Zeilen angezeigt werden. Dabei kann der Proband aufgefordert werden mitzuteilen, bis zu welcher Spalte und/oder Zeile er die Optotypen gut erkennen kann. Dies kann zu einer relativ großen Anzahl von bestimmten Visuswerten für eine ebenso großen Anzahl unterschiedlicher angelegter Refraktionen führen. Dies kann wiederum zu einer genaueren Auswertung mittels Sensitivitätsmetrik führen und somit zu einer zuverlässig bestimmten Sensitivität.

Allgemein kann der Visus auch in anderen Systemen bestimmt werden, z.B. durch eine Bestimmung von J0 und J45 und/oder Harris-Vektoren an Stelle von Achse und Zylinderstärke. Diese Technik ist z.B. aus dem Dokument "Closed Surfaces of Constant Visual Acuity in Symmetric Dioptric Power Space" von Alan Rubin et al., Optometry and Vision Science, Vol. 78, No. 10, October 2001, bekannt. Dies kann insbesondere bei Verwendung adaptiver Linsen sinnvoll genutzt werden.

Die Visusmessung kann dabei als Kontrolle der bestimmten subjektiven Refraktion verwendet werden. Dies bedeutet, dass die Probanden einen vorgegebenen Visus erreichen können oder auch nicht. Die Visusmessung kann auch dazu genutzt werden, Information über das Verhalten des Sehsystems des Probanden zu erhalten.

Dabei gibt es eine Mehrzahl an Möglichkeiten für die Visusmessung. Die Visusmessung kann z.B. mittels Optotypen erfolgen, also mittels Buchstaben, Landoltringen und/oder Ähnlichem. Es kann geprüft werden, ob der Proband die Optotypen und/oder deren Ausrichtung ganz oder teilweise erkennen kann.

Da es hierbei um eine Schwellwertbewertung gehen kann, sollte dabei sichergestellt werden, dass keine zufällige Erkennung der Optotypen vorliegt. Dies kann durch eine Wiederholung von Sehaufgaben bewirkt werden. Dabei kann ein korrektes Erkennen von 60% der Optotypen einer Menge als ein erfolgreiches Erkennen dieser Menge gewertet werden.

Weiterhin kann eine psychophysikalische Bewertung des Visus vorgenommen werden. Eine solche psychophysikalische Bewertung kann basieren auf einer Anzeige einer Sequenz von Optotypen unterschiedlicher Größe, deren Erkennen auf unterschiedliche Sehschärfen schließen lässt. Diese Sequenz kann in Abhängigkeit von Antworten des Probanden verändert werden. Ziel der Bewertung kann es sein, die Sequenz der Optotypen zu der Sehschärfe des Probanden hin konvergieren zu lassen, welche als ein Schwellwert für die Beurteilung verwendet wird. Die Variationen der Sequenzen können abhängig von den Antworten des Probanden und abhängig von der jeweils verwendeten Methode verändert werden.

Um einen dabei verwendeten Algorithmus beim Konvergieren auf die korrekte Sehschärfe zu unterstützen, kann der Proband nach der kleinsten Linie von Optotypen gefragt werden, welche er erkennen kann. Dann kann abhängig vom Ausgang überprüft werden, ob der Proband tatsächlich die ausgewählte Linie und/oder eine kleinere erkennen kann.

Der Visus kann monokular und/oder binokular ermittelt werden. Dabei handelt es sich um unterschiedliche visuelle Parameter, die alle zur Berechnung von Brillengläsern herangezogen werden können.

Die voranstehend beschriebenen Verfahren zur Visusbestimmung können dafür verwendet werden, auf eine Kontrastsensitivität hin überprüft zu werden. Dabei können Zielanzeigen mit unterschiedlichen und/oder gleichen Kontrasten angezeigt werden. Der Kontrast kann als ein Parameter in die verwendete Sensitivitätsmetrik eingehen und so berücksichtigt werden. Dadurch kann mit dem Verfahren ggf. auch die Kontrastsensitivität bestimmt werden.

Auch die psychophysikalische Bewertung des Visus kann anhand von Schärfe und/oder Kontrast vorgenommen werden. So kann der Visus über unterschiedlich große erkennbare Optoypen beispielsweise bei unterschiedlichen Kontrasten bestimmt werden.

**Durchführung von Verfahren zur Bestimmung der subjektiven Refraktion mit Erfassung von Visuswerten**

Die Verfahren zur Bestimmung der Sensitivität während der Bestimmung der subjektiven Refraktion, insbesondere das voranstehend beschriebene Ausführungsbeispiel, kann z.B. manuell durchgeführt werden mittels einer Messbrille und/oder einem Phoropter. Dabei kann ein Refraktionist die normale subjektive Refraktion durchführen und dabei vor dem Erreichen der besten Refraktion ein oder mehrmals den bei den jeweiligen Zwischenschritten eingesetzten Korrektionen erreichten Visus erfassen.

Das Verfahren kann im Rahmen einer geführten Durchführung erfolgen. Dabei wird ein Refraktionist z.B. von einem Computerprogramm durch die Bestimmung der Refraktion geführt. Dazu kann der Refraktionsablauf auf einem Computersystem implementiert sein, das den Refraktionisten auffordert, eine Phoroptereinheit und/oder eine Anzeigeeinheit entsprechend einer Vorgabe eines Algorithmus einzustellen. Rückmeldungen des Probanden können eingegeben werden und/oder automatisch erfasst werden, z.B. mittels einer Spracherkennung.

Das Verfahren kann zumindest teilautomatisch durchgeführt werden, d.h. ohne eine automatische und/oder apparative Erfassung einer Rückmeldung des Probanden. Dazu kann ebenfalls ein Computersystem genutzt werden, das mit einer Phoroptereinheit und/oder einer Anzeigeeinheit kommuniziert. Das Computersystem wirkt als eine Art Steuereinheit, welche die Phoroptereinheit und/oder die Anzeigeeinheit gemäß dem Verfahrensablauf steuert und/oder regelt. Ein Refraktionist kann dabei die Phoropter- und/oder die Anzeigeeinheit einstellen, sofern eine dieser Einheiten und/oder eine Funktion dieser Einheiten nicht durch das Computersystem gesteuert wird. Der Refraktionist kann Rückmeldungen des Probanden in das Computersystem eingeben.

Alternativ kann das Verfahren zumindest teilautomatisch mit automatisierter Erfassung von Rückmeldungen des Probanden durchgeführt werden. Hierbei können Rückmeldungen des Probanden z.B. mittels Taster, Stimmerfassung und/oder Eyetracking erfasst werden. Damit kann der Verfahrensablauf, also z.B. die Durchführung der Verfahrensschritte a) bis e), insbesondere vollständig automatisiert werden, da der Refraktionist die Rückmeldung des Probanden nicht an das Computersystem weitergeben muss. Dazu kann eine Phoroptereinheit verwendet werden, welche mit dem Computersystem kommunizieren kann. Eine dabei verwendete Anzeigeeinheit kann entweder eine festgelegte Darstellung zeigen, mit der eine Visusbestimmung möglich ist, also z.B. eine klassische Sehtafel mit Symbolen in unterschiedlichen Größen, oder sie kann ebenfalls mit dem Computersystem kommunizieren und z.B. als ein Display ausgebildet sein.

Wenngleich ein wesentlicher Aspekt der Erfindung das Erfassen des Visus vor dem Erreichen der besten Refraktion darstellt, kann in einigen Fällen ein (z.B. zusätzlicher) Visus auch nachgelagert erfasst werden, z.B. für definiert genebelte Zustände nach Bestimmung der besten Refraktion. Dies gilt insbesondere dann, wenn der Abstand zwischen der ersten und der zweiten Refraktion einen Mindestabstand unterschreitet.

Dies kann auch insbesondere dann erfolgen, wenn das Verfahren mittels eines Computerprogrammprodukts und/oder einer Vorrichtung mit einer Steuereinheit, einer Phoroptereinheit, einer Anzeigeeinheit und/oder einer Eye-Tracking-Einheit durchgeführt wird. In diesen Fällen kann die subjektive Refraktion und/oder die Sensitivität geführt oder zumindest teilweise automatisch ermittelt werden.

Dabei kann sich ein Erfassen zumindest eines Visuswertes an die Bestimmung der besten Refraktion anschließen. Dazu kann nach dem Erreichen der besten Refraktion zuerst der Visus für die beste Refraktion, und danach der Visus für eine Nebelung von beispielsweise +2 dpt sphärisch und/oder -2 dpt zylindrisch gemessen werden.

In einer Ausführungsform kann berücksichtigt werden, dass eine zu negative sphärische Wirkung einer Korrektion ggf. vom Probanden wegakkommodiert werden kann. Um dies zu vermeiden, können die angelegten Refraktionen bei der Bestimmung des Visus bevorzugt mehr Plus aufweisen als das (erwartete) Refraktionsergebnis. Dies kann während der Durchführung der Bestimmung des subjektiven Refraktionsergebnisses berücksichtigt werden.

Bei einer Verwendung von zylindrischen Korrektionen mit einem Hauptschnitt ohne Wirkung kann berücksichtigt werden, dass eine Korrektion mit gegebenem Zylinder (von z.B. +1dpt) und einem Hauptschnitt ohne Wirkung eine mittlere sphärische Wirkung des halben Zylinders hat, also im Beispiel +0,5dpt. Dies kann bei der Berechnung eines Abstandes zwischen den angelegten Refraktionen berücksichtigt werden. Wird mit Korrektionen mit einem negativen Zylinder, also z.B. -1dpt, und einem Hauptschnitt ohne Wirkung gearbeitet, beträgt eine zugehörige negative mittlere sphärische Wirkung ebenfalls der halben Zylinderkorrektion, als im Beispiel -0,5dpt. Diese negative Korrektion kann ggf. vom Probanden durch Akkommodation ausgeglichen werden und muss dann nicht mehr berücksichtigt werden. Dies kann z.B. bei nicht-presbyopen Probanden ein bevorzugtes Ausführungsbeispiel darstellen. Korrektionen wie Probegläser mit zylindrischer Wirkung ohne mittlere sphärische Wirkung können diesen Effekt vermeiden. Sie sind allerdings in der Fertigung relativ aufwändig, da jeweils ein positiver und ein negativer Hauptschnitt erforderlich ist.

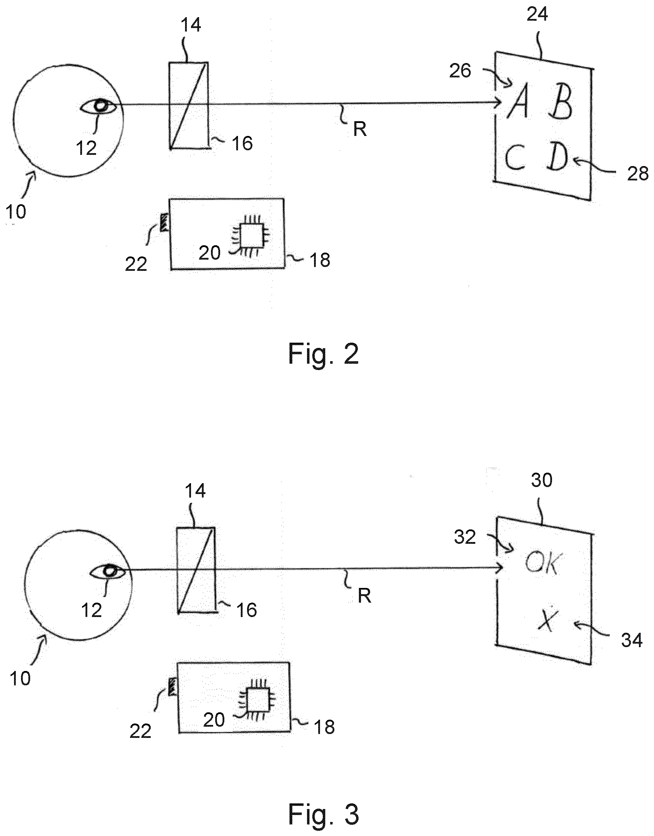

**Figur 2** zeigt in einer schematischen Darstellung eine erste Ausführungsform einer Vorrichtung zum Bestimmen einer Sensitivität zumindest eines Auges 12 eines Probanden 10 in einem ersten Anzeigezustand.

Die Vorrichtung weist eine Refraktionseinheit 14 zum Einstellen einer angelegten optischen Refraktion und/oder Korrektion für zumindest ein Auge 12 des Probanden 10 auf. Die Refraktionseinheit 14 kann z.B. als ein Phoropter ausgebildet sein und/oder einen Phoropter aufweisen. Die Vorrichtung kann weiterhin eine Eyetrackingeinheit 16 aufweisen, welche z.B. an der Refraktionseinheit 14 angeordnet sein kann, und welche dazu ausgebildet und/oder konfiguriert ist, eine Blickrichtung R und/oder eine Orientierung des zumindest einen Auges 12 des Probanden 10 zu erfassen, insbesondere während der Proband 10 ein angezeigtes Prüfbild betrachtet. Dieses Prüfbild kann auf einer Anzeigeeinheit 24 angezeigt werden und mehrere Sehzeichen 26 und 28 als Optotypen umfassen. Die Sehzeichen 26 und 28 können in Prüfbildbereichen angezeigt werden, z.B. in jedem Prüfbildbereich jeweils ein Sehzeichen 26, 28. Dazu kann jedes Sehzeichen 26, 28 mit einer zugeordneten optischen Korrektion und/oder zugeordneten angelegten Refraktion angezeigt werden.

Im gezeigten Ausführungsbeispiel blickt der Proband 10 mit seinem Auge 12 durch die Refraktionseinheit 14 entlang der Blickrichtung R auf das angeblickte Sehzeichen 26, welches in der Figur 2 als ein "A" gezeigt ist. Dabei kann die Eyetrackingeinheit 16 die Blickrichtung R erfassen. Anhand der so erfassten Blickrichtung R kann geprüft werden, dass der Proband 10 das angeblickte Sehzeichen 26 betrachtet und nicht eines der unangeblickten Sehzeichen 28, welche in der Figur 2 als "B", "C" und "D" gezeigt sind. Somit kann unterschieden werden, welches der Sehzeichen 26, 28 der Proband anblickt.

Die Vorrichtung kann weiterhin eine Steuereinheit 18 umfassen, welches eine Steuerung 20 der Refraktionseinheit 14 und/oder der Anzeigeeinheit 24 umfassen kann. Die Steuereinheit 18 kann zudem dazu ausgebildet und/oder konfiguriert sein, die von der Eyetrackingeinheit 16 erfasste Blickrichtung R auszulesen und/oder zu empfangen.

Die Vorrichtung kann weiterhin einen Auslöser 22 umfassen, welcher z.B. an der Steuereinheit 18 ausgebildet sein kann, z.B. als ein Taster. Die Steuereinheit 18 kann dazu ausgebildet und/oder konfiguriert sein, von dem Auslöser 22 generierte Signale auszulesen und/oder zu empfangen.

Die Steuereinheit 18 kann dazu ausgebildet und/oder konfiguriert sein, von der Refraktionseinheit 14 und/oder der Anzeigeeinheit 24 und/oder der Eyetrackingeinheit 16 und/oder dem Auslöser 22 generierte Signale auszuwerten.

Die Steuereinheit 18 kann weiterhin als eine Signaleinheit ausgebildet sein, welche ein Augensignal erstellt, welches Informationen zu der erfassten Blickrichtung R und/oder Orientierung des zumindest einen Auges 12 des Probanden 10 enthält. Die Steuereinheit 18 kann als eine Auswerteinheit ausgebildet sein, welche die optometrischen Parameter des Probanden 10 bestimmt unter Auswertung des Augensignals in Abhängigkeit von dem angezeigten Prüfbild.

Die Steuereinheit 18 kann weiterhin als eine Visusbestimmungseinheit ausgebildet sein, welche dazu konfiguriert ist, während des Durchführens der Bestimmung des subjektiven Refraktionsergebnisses einen ersten Visus des zumindest einen Auges 12 für eine erste angelegte Refraktion zu bestimmen und einen zweiten Visus des zumindest einen Auges 12 für eine zweite angelegte Refraktion, wobei die zweite angelegte Refraktion von der ersten angelegten Refraktion verschieden ist.

Die Steuereinheit 18 kann auch als eine Sensitivitätsermittlungseinheit ausgebildet sein, welche die Sensitivität des zumindest einen Auges 12 unter Berücksichtigung des ersten und des zweiten Visus bei der ersten und zweiten Refraktion ermittelt.

**Figur 3** zeigt in einer schematischen Darstellung eine zweite Ausführungsform einer Vorrichtung zum Bestimmen der Sensitivität eines Probanden 10 in einem zweiten Anzeigezustand. Diese Vorrichtung ist ähnlich oder gleich zu der in Fig. 2 gezeigten Vorrichtung ausgebildet, wobei gleiche Bezugszeichen gleiche oder ähnliche Merkmale kennzeichnen. Dabei kann die Steuereinheit 18 dazu ausgebildet und/oder konfiguriert sein, zusätzlich von einer Anzeigeeinheit 30 generierte Signale auszuwerten, und die Steuerung 20 kann eine Steuerung der Anzeigeeinheit 30 umfassen. Die Anzeigeeinheit 30 dieser Vorrichtung kann identisch zu der Anzeigeeinheit 24 der in Fig. 2 gezeigten Vorrichtung sein.

Die Vorrichtung weist die Anzeigeeinheit 30 auf, auf welcher zumindest ein Bestätigungsfeld 32 und/oder zumindest ein Abbruchsfeld 34 angezeigt werden kann bzw. können. Solche Bestätigungs- und/oder Abbruchfelder 32 und 34 können auch zusätzlich in den in Figur 2 gezeigten Sehzeichen 26, 28 angezeigt werden und/oder vorgesehen sein. Das Bestätigungsfeld 32 und/oder das Abbruchsfeld 34 kann als ein Betätigungsfeld ausgebildet sein, mittels welchem der Proband 10 eine Rückmeldung an die Vorrichtung geben kann.

So kann der Proband 10 z.B. gefragt werden, ob seine Blickrichtung R korrekt erfasst wurde. Dies kann über ein Audiosignal erfolgen oder z.B. über eine entsprechende Anzeige auf der Anzeigeeinheit 30 und/oder 24. Hat er die Blickrichtung R korrekt erfasst, also z.B. dass der Proband 10 soeben das angeblickte Sehzeichen 26 (z.B. "A") angeblickt hat, so kann der Proband 10 das Bestätigungsfeld 32 fixieren, falls dies korrekt ist. Wurde die Blickrichtung R nicht korrekt erfasst, kann der Proband 10 eines der Abbruchfelder 34 fixieren. Ein Fixieren des Bestätigungs- und/oder Abbruchfelds 32, 34 kann von der Eyetrackingeinheit 16 erfasst werden und von der Steuereinheit 18 ausgewertet werden.

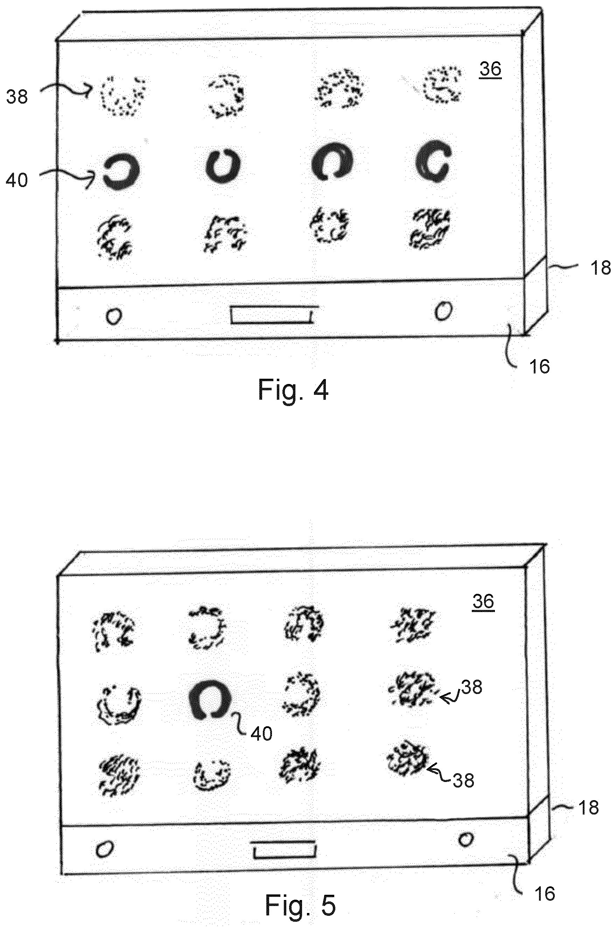

**Figur 4** zeigt in einer schematischen Darstellung ein Lichtfelddisplay 36 und/oder Lichtfeldanzeige einer Vorrichtung zur Bestimmung der Sensitivität eines Probanden. Das Lichtfelddisplay 36 kann als eine Refraktionseinheit und/oder eine Anzeigeeinheit verwendet werden. Die Vorrichtung weist eine Eyetrackingeinheit 16 auf, welche z.B. in das Lichtfelddisplay 36 integriert sein kann und/oder mit diesem verbunden sein kann. Ähnlich oder genauso wie die in den Figuren 2 und 3 gezeigten Ausführungsformen weist die Vorrichtung eine Steuereinheit 18 auf, welches zur Steuerung des Lichtfelddisplays 36 und/oder der Eyetrackingeinheit 16 ausgebildet sein kann. Die Steuereinheit 18 kann dazu ausgebildet und/oder konfiguriert sein, vom Lichtfelddisplay 36 und/oder der Eyetrackingeinheit 16 generierte Signale zu erfassen und/oder auszuwerten.

Auf dem Lichtfelddisplay 36 wird ein Prüfbild angezeigt, welches zur Bestimmung der subjektiven Refraktion des Probanden 10 dient und eine Mehrzahl von Prüfbildbereichen aufweist. Hierbei kann in jedem Prüfbildbereich z.B. zumindest ein Sehzeichen 38, 40 angezeigt werden, welche reihenweise mit derselben optischen Korrektion und/oder Wirkung und/oder Refraktion projiziert werden. Für jeden der Prüfbildbereiche kann eine zugeordnete optische Refraktion für das zumindest ein Auge 12 des Probanden 10 derart simuliert werden, dass der Eindruck entsteht, dass das zumindest eine Auge 12 das jeweilige Sehzeichen 38, 40 durch die jeweils zugeordnete optische Refraktion betrachtet. Hierbei können sich zumindest zwei der simulierten, zugeordneten optischen Refraktionen hinsichtlich ihrer optischen Wirkung voneinander unterscheiden. Die Prüfbildbereiche werden mit den zugeordneten optischen Refraktionen simultan angezeigt.

Dabei können die Sehzeichen 38, 40 jeder Reihe mit der jeweils gleichen optischen Refraktion (innerhalb der Reihe) projiziert werden. Die optischen Refraktionen mit der die Sehzeichen 38, 40 der einzelnen Reihen projiziert werden, unterschieden sich voneinander, z.B. in der verwendeten Defokuskomponente. Mittels dieser unterschiedlichen Defokuskomponenten kann die subjektiv benötigte mittlere Sphäre bestimmt werden.

Alternativ kann die optische Refraktion jeder Reihe auch die Sphärenwirkung, die Zylinderwirkung und/oder die Achse voneinander abweichen. So kann die optische Refraktion z.B. um einen festen oder variablen Betrag voneinander abweichen, z.B. um jeweils ¼ Dioptrie in der Sphäre und/oder im Zylinder. Die Refraktionen können pro Reihe um eine bestimmte Zylinderachse verdreht gegeneinander projiziert sein, z.B. jeweils um 45°.

Dadurch kann die Wahl der subjektiv besten optischen Refraktion sicher getroffen werden, da für jede optische Refraktion mehrere Sehzeichen 38, 40 zur Verfügung stehen, nämlich eine ganze Reihe Sehzeichen mit derselben optischen Refraktion. Der Proband 10 kann somit diejenige Reihe auswählen, welche mit der für den Probanden 10 subjektiv besten optischen Refraktion projiziert wird. Hierbei ist so eine sicherere Bestimmung des Visus für jede verwendete Refraktion möglich, wenn gewünscht.

**Figur 5** zeigt, ähnlich wie Figur 4, in einer schematischen Darstellung ein Lichtfelddisplay 36 als Lichtfeldanzeige einer Vorrichtung zum Bestimmen einer Sensitivität eines Probanden. Hierbei kennzeichnen gleiche Bezugszeichen gleiche oder ähnliche Merkmale.

Auf dem Lichtfelddisplay 36 werden Sehzeichen 38, 40 in Reihen und Spalten angezeigt, welche mit jeweils unterschiedlichen optischen Refraktionen projiziert werden können. Dabei können innerhalb jeder Reihe (oder alternativ Spalte) die Sehzeichen 38, 40 mit jeweils gleichen Astigmatismuskomponenten J0 der optischen Korrektur projiziert werden. Die Astigmatismuskomponenten J0 der optischen Korrektur der einzelnen Reihen (oder alternativ Spalten) unterschieden sich aber voneinander. In jeder Spalte (oder alternativ Reihe) werden dagegen die Sehzeichen 38, 40 mit jeweils gleichen Astigmatismuskomponente J45 der optischen Korrektur projiziert. Die Astigmatismuskomponenten J45 der optischen Korrektur der einzelnen Spalten (oder alternativ Reihen) unterschieden sich aber untereinander.

So kann mit den in Fig. 4 schematisch gezeigten Ansatz z.B. zunächst die Defokuskomponente und somit die mittlere Sphäre ermittelt werden. Anschließend kann z.B. mit dem in Fig. 5 schematisch gezeigten Ansatz die Astigmatismuskomponenten J0 und J45 ermittelt werden. Zusammen ergeben sich daraus Sphäre, Zylinder und Achse der subjektiv benötigten optischen Refraktion.

**Bezugszeichenliste**

| | |
|---|---|
| 1 | Kreuzzylinder |
| 2 | Griffachse |
| 3 | Zylinderachse des Pluszylinders |
| 4 | Zylinderachse des Minuszylinders |
| 5 | Griff |
| 10 | Proband |
| 12 | Auge |
| 14 | Refraktionseinheit |
| 16 | Eyetrackingeinheit |
| 18 | Steuereinheit |
| 20 | Steuerung |
| 22 | Auslöser |
| 24 | Anzeigeeinheit |
| 26 | angeblicktes Sehzeichen |
| 28 | unangeblicktes Sehzeichen |
| 30 | Anzeigeeinheit |
| 32 | Bestätigungsfeld |
| 34 | Abbruchsfeld |
| 36 | Lichtfelddisplay |
| 38 | Sehzeichen |
| 40 | Sehzeichen |
| R | Blickrichtung |

**Patentansprüche**

1. Verfahren zum Bestimmen einer Sensitivität zumindest eines Auges (12) eines Probanden (10), wobei:

   - mittels einer Refraktionseinheit eine Bestimmung eines subjektiven Refraktionsergebnisses für das zumindest eine Auge (12) des Probanden (10) durchgeführt wird;
   - während des Durchführens der Bestimmung des subjektiven Refraktionsergebnisses mittels einer Visusbestimmungseinheit ein erster Visus des zumindest einen Auges (12) für eine erste angelegte Refraktion bestimmt wird;
   - während des Durchführens der Bestimmung des subjektiven Refraktionsergebnisses mittels der Visusbestimmungseinheit ein zweiter Visus des zumindest einen Auges (12) für eine zweite angelegte Refraktion bestimmt wird, wobei die zweite angelegte Refraktion von der ersten angelegten Refraktion verschieden ist; und
   - mittels einer Sensitivitätsermittlungseinheit die Sensitivität des zumindest einen Auges unter Berücksichtigung des ersten und des zweiten Visus bei der ersten und der zweiten angelegten Refraktion ermittelt wird.

2. Verfahren nach Anspruch 1, wobei zumindest der erste Visus bestimmt wird, bevor das subjektive Refraktionsergebnis für das zumindest eine Auge (12) bestimmt ist.

3. Verfahren nach Anspruch 2, wobei als die zweite angelegte Refraktion das bestimmte subjektive Refraktionsergebnis verwendet wird und der zweite Visus für das bestimmte subjektive Refraktionsergebnis bestimmt wird.

4. Verfahren nach einem der vorangegangenen Ansprüche, wobei die Sensitivität des zumindest einen Auges (12) auf Basis einer Sensitivitätsmetrik ermittelt wird, und diese Ermittlung der Sensitivität aus Visusmessungen bei angelegten Refraktionswerten erfolgt, welche untereinander und/oder vom Refraktionsergebnis keinen speziell zur Ermittlung der Sensitivität vorgegebenen und/oder optimierten Abstand aufweisen.

**5.** Verfahren nach einem der vorangegangenen Ansprüche, wobei zur Bestimmung des subjektiven Refraktionsergebnisses unterschiedliche Optotypen (26, 28; 38, 40) angezeigt werden und während der Bestimmung des subjektiven Refraktionsergebnisses ein zu den angezeigten Optotypen (26, 28; 38, 40) gehöriger Visus als der erste und/oder zweite Visus bestimmt wird.

**6.** Verfahren nach einem der vorangegangenen Ansprüche, wobei die erste angelegte Refraktion einen Abstand von der zweiten angelegten Refraktion von zumindest einer halben Dioptrie in der Sphäre und/oder von zumindest einer Dioptrie eines Zylinders aufweist.

**7.** Verfahren nach einem der vorangegangenen Ansprüche, wobei nach dem Bestimmen des subjektiven Refraktionsergebnisses überprüft wird, ob die erste angelegte Refraktion einen vorbestimmten sphärischen und/oder zylindrischen Mindestabstand von der zweiten angelegten Refraktion aufweist und für den Fall, dass dieser vorbestimmte Mindestabstand unterschritten ist, ein dritter Visus für eine dritte angelegte Refraktion bestimmt wird, welche zumindest um den vorbestimmten Mindestabstand von der ersten und/oder zweiten angelegten Refraktion beabstandet ist, und die Sensitivität des zumindest einen Auges unter Berücksichtigung des dritten Visus bei der dritten angelegten Refraktion ermittelt wird.

**8.** Verfahren nach einem der vorangegangenen Ansprüche, wobei die Sensitivität des zumindest einen Auges (12) auf Basis eines linearen Modells ermittelt wird, bei welchem eine Abhängigkeit der Sensitivität für einen zylindrischen Refraktionsabstand von der Sensitivität für einen sphärischen Refraktionsabstand angenommen wird.

**9.** Verfahren nach einem der vorangegangenen Ansprüche, wobei ein dritter Visus des zumindest einen Auges (12) für eine dritte angelegte Refraktion bestimmt wird, und wobei die Sensitivität des zumindest einen Auges (12) unter Berücksichtigung des ersten, zweiten und dritten Visus bei der ersten, zweiten und dritten angelegten Refraktion auf Basis eines bilinearen Modells ermittelt wird.

**10.** Verfahren nach einem der vorangegangenen Ansprüche, wobei die Sensitivität ohne Berücksichtigung des bestimmten subjektiven Refraktionsergebnisses ermittelt wird.

**11.** Verfahren nach einem der vorangegangenen Ansprüche, wobei die Bestimmung des subjektiven Refraktionsergebnisses mittels einer Refraktionseinheit (14; 36) durchgeführt wird und/oder die Visusbestimmung mittels dem Probanden (10) angezeigten Optotypen (26, 28; 38, 40) erfolgt.

**12.** Verfahren nach einem der vorangegangenen Ansprüche, wobei das Bestimmen des subjektiven Refraktionsergebnisses, das Bestimmen des ersten und zweiten Visus, und/oder das Bestimmen der Sensitivität softwaregestützt und/oder zumindest teilautomatisch erfolgt.

**13.** Verfahren nach einem der vorangegangenen Ansprüche, wobei der erste Visus und/oder der zweite Visus monokular und/oder binokular bestimmt wird;
und/oder
wobei die ermittelte Sensitivität dazu verwendet wird, zumindest ein individuelles Brillenglas für den Probanden (10) zu erstellen.

**14.** Vorrichtung zum Bestimmen einer Sensitivität zumindest eines Auges (12) eines Probanden (10) mit:

- einer Refraktionseinheit (14; 36) zum Bestimmen eines subjektiven Refraktionsergebnisses für das zumindest eine Auge (12) des Probanden (10);
- einer Visusbestimmungseinheit, welche dazu konfiguriert ist, während des Durchführens der Bestimmung des subjektiven Refraktionsergebnisses einen erster Visus des zumindest einen Auges (12) für eine erste angelegte Refraktion zu bestimmen und einen zweiten Visus des zumindest einen Auges (12) für eine zweite angelegte Refraktion, wobei die zweite angelegte Refraktion von der ersten angelegten Refraktion verschieden ist; und
- eine Sensitivitätsermittlungseinheit, welche die Sensitivität des zumindest einen Auges (12) unter Berücksichtigung des ersten und des zweiten Visus bei der ersten und zweiten Refraktion ermittelt.

**15.** Computerprogrammprodukt umfassend computerlesbare Programmteile, welche geladen und ausgeführt eine Vorrichtung nach Anspruch 14 dazu bringen, ein Verfahren nach einem der Ansprüche 1 bis 13 durchzuführen, wobei das Computerprogrammprodukt zumindest die folgenden Einheiten zumindest teilweise steuert und/oder regelt:

- die Refraktionseinheit (14; 36);
- die Visusbestimmungseinheit; und
- die Sensitivitätsermittlungseinheit;

und wobei das Computerprogrammprodukt insbesondere eine oder beide der folgenden Einheiten zumindest teilweise steuert und/oder regelt:

- eine Steuerung (18); und/oder
- eine Brillenglasdatenerstellungseinheit zum Erstellen zumindest eines individuellen Brillenglases und/oder zum Berechnen zumindest einer Brillenglasfläche aus den erfassten Messdaten.

## Claims

1. Method for determining the sensitivity of at least one eye (12) of a test subject (10), wherein:

   - a subjective refraction result for the at least one eye (12) of the test subject (10) is determined by means of a refraction unit;
   - while the subjective refraction result is being determined by means of a visual acuity determination unit, a first visual acuity of the at least one eye (12) is determined for a first refraction applied;
   - while the subjective refraction result is being determined by means of the visual acuity determination unit, a second visual acuity of the at least one eye (12) is determined for a second applied refraction, wherein the second applied refraction is different from the first applied refraction; and
   - the sensitivity of the at least one eye is determined by means of a sensitivity determination unit, taking into account the first and second visual acuity values for the first and second applied refractions.

2. Method according to claim 1, wherein at least the first visual acuity is determined before the subjective refraction result for the at least one eye (12) is determined.

3. Method according to claim 2, wherein the determined subjective refraction result is used as the second refraction applied and the second visual acuity is determined for the determined subjective refraction result.

4. Method according to one of the preceding claims, wherein the sensitivity of the at least one eye (12) is determined on the basis of a sensitivity metric, and this determination of sensitivity is made from visual acuity measurements at applied refraction values which do not have a distance between each other and/or from the refraction result which is specifically predetermined and/or optimized for determining sensitivity.

5. Method according to one of the preceding claims, wherein different optotypes (26, 28; 38, 40) are displayed to determine the subjective refraction result and, during the determination of the subjective refraction result, an optotype (26, 28; 38, 40) corresponding to the displayed optotypes (26, 28; 38, 40) are displayed to determine the subjective refraction result and, during the determination of the subjective refraction result, a visual acuity corresponding to the displayed optotypes (26, 28; 38, 40) is determined as the first and/or second visual acuity.

6. Method according to one of the preceding claims, wherein the first refraction applied has a deviation from the second refraction applied of at least half a diopter in the sphere and/or at least one diopter of a cylinder.

7. Method according to one of the preceding claims, wherein after determining the subjective refraction result, it is checked whether the first applied refraction has a predetermined spherical and/or cylindrical minimum distance from the second applied refraction and, if this predetermined minimum distance is not reached, a third visual acuity is determined for a third refraction applied, which is spaced at least by the predetermined minimum distance from the first and/or second refraction applied, and the sensitivity of the at least one eye is determined taking into account the third visual acuity in the third refraction applied.

8. Method according to one of the preceding claims, wherein the sensitivity of the at least one eye (12) is determined on the basis of a linear model in which a dependence of the sensitivity for a cylindrical refractive power on the sensitivity for a spherical refractive power is assumed.

9. Method according to one of the preceding claims, wherein a third visual acuity of the at least one eye (12) is determined for a third refraction applied, and wherein the sensitivity of the at least one eye (12) is determined on the basis of a bilinear model, taking into account the first, second, and third visual acuity during the first, second, and third refractions applied.

10. Method according to one of the preceding claims, wherein the sensitivity is determined without taking into account the determined subjective refraction result.

11. Method according to one of the preceding claims, wherein the subjective refraction result is determined by means of a refraction unit (14; 36) and/or the visual acuity is determined by means of optotypes (26, 28; 38, 40) displayed to the test subject (10).

12. Method according to one of the preceding claims, wherein the determination of the subjective refraction result, the determination of the first and second visual acuity, and/or the determination of the sensitivity is performed with the aid of software and/or at least semi-automatically.

13. Method according to one of the preceding claims, wherein the first visual acuity and/or the second visual acuity is determined monocularly and/or binocularly;
and/or
wherein the determined sensitivity is used to create at least one individual eyeglass lens for the test subject (10).

14. Device for determining a sensitivity of at least one eye (12) of a test subject (10), comprising:

    - a refraction unit (14; 36) for determining a subjective refraction result for the at least one eye (12) of the test subject (10);
    - a visual acuity determination unit which is configured to determine, during the determination of the subjective refraction result, a first visual acuity of the at least one eye (12) for a first applied refraction and a second visual acuity of the at least one eye (12) for a second applied refraction, wherein the second applied refraction is different from the first applied refraction; and
    - a sensitivity determination unit which determines the sensitivity of the at least one eye (12) taking into account the first and second visual acuity values in the first and second refractions.

15. Computer program product comprising computer-readable program parts which, when loaded and executed, cause a device according to claim 14 to perform a method according to one of claims 1 to 13, wherein the computer program product at least partially controls and/or regulates the following units:

    - the refraction unit (14; 36);
    - the visual acuity determination unit; and
    - the sensitivity determination unit;

and wherein the computer program product in particular controls and/or regulates at least one or both of the following units at least in part:

    - a controller (18); and/or
    - a lens data creation unit for creating at least one individual lens and/or for calculating at least one lens surface from the measured data.

## Revendications

1. Procédé pour déterminer une sensibilité d'au moins un oeil (12) d'un sujet (10), dans lequel :

    - une détermination d'un résultat de réfraction subjectif pour l'au moins un œil (12) du sujet (10) est effectuée au moyen d'une unité de réfraction ;
    - pendant l'exécution de la détermination du résultat de réfraction subjectif, on détermine, au moyen d'une unité de détermination de l'acuité visuelle, une première acuité visuelle de l'au moins un œil (12) pour une première réfraction appliquée ;
    - pendant l'exécution de la détermination du résultat de la réfraction subjective au moyen de l'unité de détermination de l'acuité visuelle, une deuxième acuité visuelle du au moins un œil (12) est déterminée pour une deuxième réfraction appliquée, la deuxième réfraction appliquée étant différente de la première réfraction appliquée ; et

- au moyen d'une unité de détermination de la sensibilité, on détermine la sensibilité de l'au moins un œil en tenant compte de la première et de la deuxième acuité visuelle pour la première et la deuxième réfraction appliquées.

2. Procédé selon la revendication 1, dans lequel au moins la première acuité visuelle est déterminée avant que le résultat de réfraction subjective pour l'au moins un œil (12) ne soit déterminé.

3. Procédé selon la revendication 2, dans lequel le résultat de réfraction subjectif déterminé est utilisé comme la deuxième réfraction appliquée et la deuxième acuité visuelle est déterminée pour le résultat de réfraction subjectif déterminé.

4. Procédé selon l'une des revendications précédentes, dans lequel la sensibilité d'au moins un œil (12) est déterminée sur la base d'une métrique de sensibilité, et cette détermination de la sensibilité s'effectue à partir de mesures de l'acuité visuelle pour des valeurs de réfraction appliquées qui ne présentent pas entre elles et/ou par rapport au résultat de réfraction une distance spécialement prédéfinie et/ou optimisée pour la détermination de la sensibilité.

5. Procédé selon l'une des revendications précédentes, dans lequel différents optotypes (26, 28 ; 38, 40) sont affichés pour déterminer le résultat subjectif de la réfraction et, pendant la détermination du résultat subjectif de la réfraction, une acuité visuelle appartenant aux optotypes affichés (26, 28 ; 38, 40) est déterminée comme la première et/ou la deuxième acuité visuelle.

6. Procédé selon l'une des revendications précédentes, dans lequel la première réfraction appliquée présente une distance par rapport à la deuxième réfraction appliquée d'au moins une demi-dioptrie dans la sphère et/ou d'au moins une dioptrie d'un cylindre.

7. Procédé selon l'une des revendications précédentes, dans lequel, après avoir déterminé le résultat de la réfraction subjective, on vérifie si la première réfraction appliquée présente une distance minimale sphérique et/ou cylindrique prédéterminée par rapport à la deuxième réfraction appliquée et, dans le cas où cette distance minimale prédéterminée n'est pas atteinte, une troisième acuité visuelle est déterminée pour une troisième réfraction appliquée, qui est espacée de la première et/ou de la deuxième réfraction appliquée au moins de la distance minimale prédéterminée, et la sensibilité d'au moins un œil est déterminée en tenant compte de la troisième acuité visuelle pour la troisième réfraction appliquée.

8. Procédé selon l'une des revendications précédentes, dans lequel la sensibilité d'au moins un œil (12) est déterminée sur la base d'un modèle linéaire, dans lequel on suppose une dépendance de la sensibilité pour une distance de réfraction cylindrique par rapport à la sensibilité pour une distance de réfraction sphérique.

9. Procédé selon l'une des revendications précédentes, dans lequel une troisième acuité visuelle de l'au moins un œil (12) est déterminée pour une troisième réfraction appliquée, et dans lequel la sensibilité de l'au moins un œil (12) est déterminée sur la base d'un modèle bilinéaire en tenant compte de la première, de la deuxième et de la troisième acuité visuelle pour la première, la deuxième et la troisième réfraction appliquée.

10. Procédé selon l'une des revendications précédentes, dans lequel la sensibilité est déterminée sans tenir compte du résultat de réfraction subjectif déterminé.

11. Procédé selon l'une des revendications précédentes, dans lequel la détermination du résultat de réfraction subjectif est effectuée au moyen d'une unité de réfraction (14 ; 36) et/ou la détermination de l'acuité visuelle est effectuée au moyen d'optotypes (26, 28 ; 38, 40) indiqués au sujet (10).

12. Procédé selon l'une des revendications précédentes, dans lequel la détermination du résultat de réfraction subjectif, la détermination de la première et de la deuxième acuité visuelle, et/ou la détermination de la sensibilité s'effectue à l'aide d'un logiciel et/ou de manière au moins partiellement automatique.

13. Procédé selon l'une des revendications précédentes, dans lequel la première acuité visuelle et/ou la deuxième acuité visuelle est déterminée de manière monoculaire et/ou binoculaire ;
et/ou
la sensibilité déterminée étant utilisée pour créer au moins un verre de lunettes individuel pour le sujet (10).

**14.** Dispositif pour déterminer une sensibilité d'au moins un œil (12) d'un sujet (10), comprenant :

- une unité de réfraction (14 ; 36) pour déterminer un résultat de réfraction subjectif pour l'au moins un œil (12) du sujet (10) ;
- une unité de détermination de l'acuité visuelle configurée pour déterminer une première acuité visuelle dudit au moins un oeil (12) pour une première réfraction appliquée et une seconde acuité visuelle dudit au moins un oeil (12) pour une seconde réfraction appliquée, ladite seconde réfraction appliquée étant différente de ladite première réfraction appliquée, pendant l'exécution de la détermination du résultat de réfraction subjectif ; et
- une unité de détermination de la sensibilité qui détermine la sensibilité de l'au moins un oeil (12) en tenant compte de la première et de la deuxième acuité visuelle à la première et à la deuxième réfraction.

**15.** Produit programme d'ordinateur comprenant des parties de programme lisibles par ordinateur qui, chargées et exécutées, amènent un dispositif selon la revendication 14 à mettre en œuvre un procédé selon l'une quelconque des revendications 1 à 13, ledit produit programme d'ordinateur commandant et/ou régulant au moins partiellement les unités suivantes :

- l'unité de réfraction (14 ; 36) ;
- l'unité de détermination de l'acuité visuelle ; et
- l'unité de détermination de la sensibilité ;

et dans lequel le produit programme d'ordinateur commande et/ou régule en particulier au moins partiellement l'une ou les deux des unités suivantes :

- une commande (18) ; et/ou
- une unité de création de données de verres de lunettes pour créer au moins un verre de lunettes individuel et/ou pour calculer au moins une surface de verre de lunettes à partir des données de mesure saisies.

Fig. 1A

Fig. 1B

Fig. 2

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2499534 B1
- US 20200241320 A1
- DE 102017007663 A1
- DE 102011120973 A1

**In der Beschreibung aufgeführte Nicht-Patentliteratur**

- **D. METHLING**. Bestimmung von Sehhilfen. Ferdinand Enke Verlag, 1996
- **APPLEGATE, R.A** ; **SARVER, E.J** ; **KHEMSARA, V.** Are all aberrations equal?. *J Refract Surg*, 2002, vol. 18, S556-S562
- **ATCHISON et al.** Blur limits for defocus, astigmatism and trefoil. *VisionResearch.*, 2009
- **R. BLENDOWSKE**. *Unaided Visual Acuity and Blur: A Simple Model, Optometry and Vision Science*, 2015, vol. 92 (6)
- **A. RUBIN** ; **W. F. HARRIS**. *Closed Surfaces of Constant Visual Acuity in Symmetric Dioptric Power Space, Optometry and Vision Science*, 2001, vol. 78 (10)
- **ALAN RUBIN et al.** Closed Surfaces of Constant Visual Acuity in Symmetric Dioptric Power Space. *Optometry and Vision Science*, October 2001, vol. 78 (10)